Europäisches Patentamt

**European Patent Office**

Office européen des brevets

(19)

(11) Publication number:

**0 060 052**

**A1**

(12)

# EUROPEAN PATENT APPLICATION

(21) Application number: 82300914.7

(22) Date of filing: 23.02.82

(51) Int. Cl.³: **C 12 N 11/04**
**C 12 N 11/08**

(30) Priority: 27.02.81 JP 26887/81
27.02.81 JP 26888/81
07.04.81 JP 51096/81

(43) Date of publication of application:
15.09.82 Bulletin 82/37

(84) Designated Contracting States:
BE CH DE FR GB IT LI NL

(71) Applicant: RESEARCH ASSOCIATION FOR
PETROLEUM ALTERNATIVE DEVELOPMENT
4-2, 1-chome Uchikanda
Chiyoda-ku Tokyo(JP)

(72) Inventor: Nambu, Masao
447-17 Honmoku-Motomachi Naka-ku
Yokohama-shi Kanagawa-ken(JP)

(72) Inventor: Sakayanagi, Sadao
895-69 Tomioka-cho Kanazawa-ku
Yokohama-shi Kanagawa-ken(JP)

(74) Representative: Ritter, Stephen David et al,
Mathys & Squire 10 Fleet Street
London EC4Y 1AY(GB)

(54) Method of immobilizing live microorganisms.

(57) A method is provided for immobilizing a live microorganism in a solid matrix. According to the invention a 1 to 25 weight % aqueous solution of a polyvinyl alcohol having a degree of hydrolysis not less than 95 mol% and a viscosity-average polymerization degree of not less than 1,500 is prepared, said live microorganism are added into said aqueous polyvinyl alcohol solution, the resulting aqueous suspension poured into a desired shape of a vessel or a mold, the aqueous suspension is frozen at a temperature lower than $-6°C$, the resulting molded article is vacuum dehydrated without thawing it to a dehydration percentage not lower than 5 weight %, and the dehydrated article thawed.

EP 0 060 052 A1

## METHOD OF IMMOBILIZING LIVE MICROORGANISMS

Background of the Invention

The present invention relates to a method of immobilizing live microorganisms.

Methods of immobilizing live microorganisms into polyvinyl alcohols, polyacrylamides, alginic acid, carrageenan, agar and clay minerals are already known, but all of those known methods have drawbacks as summarized below and the development of a more effective immobilization method has been desired.

(1) There is known a method of obtaining a film containing a live microorganism by mixing the microorganism into an aqueous polyvinyl alcohol solution and then air-drying the mixture. However, the film thereby obtained is inferior in water resistance and is weak and its microorganism entrapping capacity is low. It has been proposed to support (reinforce) this film with a nylon cloth or the like, but most of the above-mentioned drawbacks are still not overcome. In addition, since the polyvinyl alcohol films are poor in permeability for carbon sources, nitrogen sources and inorganic matters required for the activity of microorganisms, the activity of live microorganisms immobilized therein is deteriorated [see Japanese Patent Laying Open Print No.145592/1977, Japanese Patent Publication No.35415/1980, and Plastic Zairyo Koza 14, pp.135 and 133 (1970), Nikkan Kogyo Shinbunsha].

(2) It is also well known to mix an aqueous polyvinyl alcohol solution with a microorganism and then apply cobalt 60 ($\gamma$-ray) to the mixture to crosslink and gel the polyvinyl alcohol.

In this case, however, a bad influence upon the microorganism is unavoidable even in combination with a protective substance against radiation damage such as glycerin. Besides, the irradiation cost is high and the resultant gel is weak, often requiring a secondary hardening treatment with a chemical reagent [see Biotech. Bioeng., 15, 607 (1973), and Hakko to Kogyo, 35, 92 (1977)].

(3)   It has long been well known that an aqueous polyvinyl alcohol solution gels upon mixing with boric acid or an aqueous boric acid solution. It has been tried to immobilize microorganisms by utilizing this principle. But the gel formed from a polyvinyl alcohol having a high degree of hydrolysis is weak and difficult to mold. This drawback is somewhat remedied by using a polyvinyl alcohol having a low degree of hydrolysis, but since the gel is sticky, its molded articles, for example, its cut pieces, are difficult to retain their shape (see Japanese Patent Laying Open Print No.135295/1979 and Japanese Patent Publication No.51552/1980).

(4)        It has also been proposed to prepare a composite polyvinyl alcohol - silicic acid yeast film by adding an acid to an aqueous suspension containing a polyvinyl alcohol, tetraethyl silicate and a microorganism  to produce composite sol, then followed by air-drying. But this film is also weak.

In this case, even if freeze-drying is applied after addition of the acid, the mechanical strength of the resultant film is rather deteriorated to the extent that the molding of the film is almost impossible. Anyhow, such proposed method involves the step of adjusting the suspension of the microorganism to a pH value not more than 3.0 by adding an acid, so a bad influence upon the microorganism is often not negligible (see Japanese Patent Publications Nos.11311/1980 and 30358/1980).

(5)    It has also been proposed to immobilize an enzyme by gelling (freeze-solidifying) an aqueous solution of a polyvinyl alcohol and the enzyme at a low temperature (see Japanese Patent Laying Open Print No.52296/1975). But the gel formed by a mere freeze-solidifying treatment does not exhibit elasticity and is very low in both tensile and compressive strengths. In case air-drying is applied after freeze-solidifying and thawing, there merely is obtained a weak film as in the case of the above (1).

Furthermore, in case dehydration is made under reduced pressure after freeze-solidifying and thawing, the thawed solution bubbles so vigorously that it often becomes impossible to continue the operation. Moreover, a long time is needed, and even after the dehydration the gel little exhibits elasticity. Additionally, the gel is fragile, whitely turbid and less hydrous.

(6)    It is also well known to immobilize a live microorganism by deoxidating a mixed aqueous suspension of acrylamide, N,N'-methylenebisacrylamide and the live microorganism and then applying radiation or adding a radical forming agent to the suspension. But it is also well known that the residual monomer in the resultant gel is very poisonous. Not only the damage to the live microorganism caused by such residual monomer and radiation or the radical is conspicuous, but also the mechanical strength of the gel is so low that a secondary hardening treatment is often required [see Biotech. Bioeng., 20, 1267 (1978)].

(7)    It is further well known to immobilize a live microorganism by subjecting a mixed aqueous suspension of sodium alginate and the live microorganism to the action of calcium ion or aluminum ion (thereby gelling it). But the gel thus formed is poor in its mechanical strength (particularly compressive strength) and is often affected and destroyed by a buffer solution (pH 7) of potassium dihydrogenphosphate [see Biotech. Bioeng., $\underline{19}$, 387 (1977)].

(8)      Like the aforesaid alginate (poly-1,4-$\beta$-D-mannuronate, L-glucuronate copolymer), the following are also mentioned as typical examples of natural polysaccharides: locust been gum (D-mannose, D-galactose system), pectin (poly-1,4-$\alpha$-D-galacturonic acid system), agar (acidic sulfate of polygalactose), carrageenan (also acidic sulfate of polygalactose). Also with respect to these natural polysaccharides, like the foregoing alginate, various attempts have been made for utilizing them as immobilizing carriers for live microorganisms. However, as a drawback common to these natural polysaccharides, their mechanical strength is not sufficiently high and an auxiliary (secondary) hardening treatment is often required. Moreover, aqueous polysaccharide solutions often solidify (gel) at room temperature and heat must be applied to above 40-50°C when mixing them with live microorganisms thus resulting in great damage to many live microorganisms. Also in the above-mentioned auxiliary (secondary) hardening treatment, the heating to say 50°C or higher or the use of a chemical reagent, a solvent, etc. would damage the live microorganisms. Furthermore, natural polysaccharides, as often observed, easily rot if contaminated by indoor saprophytes. For example, if an aqueous solution of gum arabic (L-rhamnopyranose, L-arabofuranose, galactopyranose, glucuronic

acid system) is put in a narrow necked glass bottle and allowed to stand in a room while the bottle is opened, usually a grayish white mould grows vigorously in 4 or 5 days even in winter. Furthermore, there have been reported examples in which the quality of natural polysaccharides is not always uniform, depending on the place of origin or the time of harvest. Also with respect to their chemical compositions and chemical structures, there are many unknown points. Agar has long been used in laboratories for the immobilization (as a culture carrier) of live microorganisms, and carrageenan which is most similar in chemical structure to agar has also been proposed as a carrier for live microorganisms and proved to function similarly. However, the foregoing drawbacks are encountered also in these polysaccharides, and the development of a superior carrier has been desired [see Enzyme Microb. Technol., 1, 95 (1979), and Kobunshi, 29, 238 (1980)].

(9)    Physical adsorption of microorganisms or enzymes to bentonite, kaolin, acid clay, active white earth, active carbon, calcium phosphate, alumina, silica gel, zeolite and pumice has also been tried. In such a physical adsorption, however, it has often been pointed out that the adsorption capacity is low and the separation after immobilization (adsorption) is noticeable [see Kobunshi, 16, 823 (1967); Shokubai, 14, 71 (1972); Kagaku Kogaku, 38, 357 (1974); Kagaku Kogyo, 1975, 1206; Maku, 3 (5) 339 (1978); Biotech. Bioeng., 15, 69 (1973); Hakko Kyokaishi, 23, 267 (1965), and Yuki Gosei Kyokaishi, 28, 471 (1970)].

6

0060052

## Summary of the Invention

It is an object of the present invention to overcome the foregoing disadvantages of the prior arts.

It is another object of the present invention to provide a method of producing a hydrogel which is water-insoluble and has a high water content and which is very elastic and mechanically strong and is stable over a long period, and at the same time embedding (entrapping) a live microorganism into the hydrogel almost completely without any damage thereto.

Other objects and advantages of the present invention will become apparent from the following description.

The above-mentioned objects of this invention are attained by the method comprising the steps: preparing a 1 to 25 wt.% aqueous solution of a polyvinyl alcohol having a degree of hydrolysis not less than 95 mol% and a viscosity-average polymerization degree of not less than 1,500, adding a live microorganism into said aqueous polyvinyl alcohol solution, pouring the resulting aqueous suspension into a desired shape of a vessel or a mold, freeze-molding the aqueous suspension at a temperature lower than -6°C, vacuum-dehydrating the resulting molded article without thawing it to a dehydration percentage not lower than 5 wt.%, thawing the dehydrated article, and if required immersing the thawed, dehydrated article in water until its water content reaches 20 to 92 wt.% (on a wet body basis).

In the method of the present invention clay minerals may be co-existent in the aqueous polyvinyl alcohol solution.

According to the present invention, gel is formed in the course of freeze-molding and dehydrating a mixed suspension of an aqueous polyvinyl alcohol solution or an aqueous suspension thereof with clay minerals and a live microorganism, and almost all quantity of the live microorganism is embedded (entrapped) in the gel. In this immobilizing step the present invention uses no acid, alkali, radiation, radical forming agent, organic solvent, or reagent, nor does it require a secondary hardening treatment. Consequently, the microorganism is little damaged (of course escapes death) and entrapped as a live microorganism, so its intrinsic activity is retained as it is.

In the conventional method for immobilizing a microorganism using a reagent or $\gamma$-ray, the total quantity or most of the microorganism often die.

On the other hand, the present invention permits immobilization of live microorganisms without damaging them with $\gamma$-ray or reagents (or reaction solvents).

The immobilizing carrier (gel) used in the present invention is a rubbery, elastic body having a high water content and being superior in permeability for carbon source, nitrogen source, oxygen gas, carbon dioxide gas and other inorganic matters. Besides, its mechanical strength is high.

It has long been well known that an aqueous polyvinyl alcohol solution often becomes more viscous or gels on storage for one day to one week at 0° to 30°C. However, as is often the case with the foregoing natural polysaccharides, the gel thus formed is fragile like agar, and what is worse, it dissolves on stirring merely vigorously or with water added or on warming a little. On the other hand, the gel of the present invention is insoluble in water or warm water and thus is quite different from the aforesaid conventional gel. This indicates that the present
invention provides a novel gel quite different from gels based on
conventional knowledges such as known gelation of an aqueous
polyvinyl alcohol solution or the foregoing chemical gelation of
an aqueous polyvinyl alcohol solution.

In the present invention, polyvinyl alcohols and
clay minerals may be used together. Regarding this combination
of polyvinyl alcohols and clay minerals, certain effects different
from the combination effect in the present invention are already
well know. That is, an attempt to modify the surface soil of a
stadium into a soil from which dust is relatively difficult to
rise by scattering a polyvinyl alcohol (as a dilute aqueous
solution) over the said surface soil, an attempt to improve the
water permeability or water retaining property of the soil of
fields by scattering a small amount of polyvinyl alcohol (as a
dilute aqueous solution) over the fields, and the technique of
promoting the flocculation and precipitation of clay (colloidal
particles) in a muddy water by adding a small amount of polyvinyl
alcohol into the muddy water, are also well known. In these
cases, it has been confirmed that by the action of polyvinyl
alcohol the soil particles undergo changes in their state of
dispersion or in their size. At least in appearance, however,
the soil is a mere soil and is very fragile and easily collapses
even in the state of dry powder, not to mention in water. It
is also well known that a hard film is obtainable by adding a
clay into an aqueous polyvinyl alcohol solution followed by
heat-drying. But this film is a stiff film poor in water
absorbing property. The gel of the present invention is entirely different from these conventional polyvinyl alcohol - clay composites known as soil modifiers or hard films.

It is also well known that clay minerals such as montmorillonite, vermiculite and synthetic taeniolite adsorb enzymes (or polyvinyl alcohols). But this proved to be a phenomenon such that enzymes get in between crystal layers of the clay minerals, and the spacing of the crystal layers is considered to be 1.5 to 6 nm at most. Thus, the spacing of such adsorbing crystal layers does not reach even the thickness (20-30 nm) of the cell wall of microorganisms. It is apparent that microorganisms can never get in that space. This fact clearly shows that the present invention provides a microorganism immobilizing technique entirely different from the conventional knowledge on the interaction between clay minerals and enzymes or between clay minerals and polyvinyl alcohols.

Detailed Description of the Preferred Embodiments

The present invention employs polyvinyl alcohols having a degree of hydrolysis not less than 95 mol%, preferably not less than 97 mol%. Even if polyvinyl alcohols having a degree of hydrolysis of 80 to 88 mol% considered to be preferable for the soil improvement previously noted are used in the present invention, there merely is produced a weak gel like mud.

In the present invention, moreover, polyvinyl alcohols having a viscosity-average polymerization degree of not less than 1,500 are used. As the polymerization degree of polyvinyl alcohol decreases, the mechanical strength of the resulting gel decreases as well, so it is preferable in the present invention to use commercially available products of high polymerization degree (about 1,700-2,600).

According to the method of the present invention, first an aqueous solution of a polyvinyl alcohol is prepared. The concentration of polyvinyl alcohol is not specially limited. For example, it may be 1 to 25 wt.%, preferably 3 to 20 wt.%, more preferably 7 to 15 wt.%. Its concentration can be further increased, but in this case the viscosity of the aqueous solution at room temperature reaches as high as 10,000 cP or more, or the aqueous solution in storage may undergo an increase in its viscosity, or its gelation may take place. Therefore, its handling is a little difficult. (The gel formed during storage of the aqueous polyvinyl alcohol solution is water-soluble and fragile like agar, quite different from the gel of the present invention.) In case the concentration of the aqueous polyvinyl alcohol solution is set below 1 wt.%, the time required for dehydration (drying) as will be described later becomes longer, the cost (of dehydrating power) is increased and there is obtained only a weak gel.

The amount of polyvinyl alcohol usually should be not less than one-fifth of the amount of clay minerals as will be described later. If this proportion is smaller than one-fifth, for example, at one-tenth, the resulting gel will be inferior in its mechanical strength. In the soil improvement as referred to in the previous discussion, the mixing ratio of polyvinyl alcohol to clay minerals is 1/1000 to 1/100, but under such a condition the microorganism immobilizing carrier (gel) of the present invention will never be obtainable.

Clay minerals used in the invention are laminated structure type clay minerals having a three-layer type (2:1 type) composite layer as a basic unit, typical of which are montmorillonite, vermiculite, illite, pyrophyllite and talc. Bentonite, which is known as a general clay, is inexpensive and that easily available. It is an aggregate of colloidal particles consisting mainly of montmorillonite produced by the weathering of tuff, rhyolite, etc. in various districts of Japan such as Hokkaido, Akita, Yamagata, Niigata, Gunma and Shimane. Montmorillonite as the main component of bentonite is also called smectite and it is of a laminated structure having a three-layer (2:1 type) composite layer as a basic unit consisting of silica (tetrahedral structure), alumina, i.e. gibbsite (octahedral structure) and silica (tetrahedral structure). Besides, a part of aluminum which constitutes the composite layer is substituted by magnesium, and among the layers there exists water and cations such as sodium, potassium, calcium, lithium, strontium, barium, aluminum, cesium, magnesium, ammonium or hydrogen. Typical structure of montmorillonite is represented, for example, by $(Al_{5/3}Mg_{1/3})$ $Si_4O_{10}(OH)_2 \cdot XH_2O(K, Na, Ca, H, NH_4, Mg, Al, Li, Cs, Sr, Ba)_y$.

As homologues obtained by substituting the main constituent elements (aluminum and silicon) of this composite layer by other elements, there are well known nontronite (iron-substituted), hectorite (magnesium-substituted), saponite (magnesium-substituted), beidellite (aluminum-substituted), sauconite (iron-, magnesium- and zinc-substituted), and volkonskoite (chromium-substituted). These are also called montmorillonite group minerals and are often found in the foregoing bentonite.

Bentonite contains about 50% to about 85% of montmorillonite and the foregoing montmorillonite group minerals. In addition, quartz, feldspar, zeolite, kaolin, illite (mica) and cristobalite are also mixed therein. Therefore, the composition (wt.%) of bentonite is not definite, but the following is a general composition: $SiO_2$ 42-65, $Al_2O_3$ 14-28, $H_2O$ 11-23, MgO 1-25, $Fe_2O_3$ 0-4, $Na_2O$ 0-3.5, CaO 0-3, $K_2O$ 0.1-0.7, $TiO_2$ 0-0.7, FeO 0-9.3, $P_2O_5$ 0-0.04.

The Pharmacopoeia of Japan defines tests concerning the swelling property of bentonite and its gel forming ability (porridge-like, composite magnesium oxide - bentonite gel forming ability), but the commercially available bentonite usually does not come up to this standard. In the present invention, however, even such a bentonite may be used as clay minerals without any trouble. Bentonite is often treated with an aqueous solution of sodium chloride, sodium hydroxide, sodium carbonate, sodium nitrate, ammonium hydroxide, sodium pyrophosphate, sodium hexametaphosphate (a low grade polymer of sodium metaphosphate), hydrochloric acid, sulfuric acid or citric acid in order to enhance its swelling property, dispersibility and specific surface area. In the present invention, it is not particularly necessary to treat bentonite in such a manner, but the so-treated bentonite may also be used without any trouble.

In the present invention, in addition to bentonite there may be used acid clay (kambara earth), active white earth, Fuller's earth, Florida earth and Georgia earth as montmorillonite clay minerals. These clay minerals contain fairly large amounts of kaolinite which belongs to neither montmorillonite nor montmorillonite-like three-layer type clay minerals as will be described later, and allophane which is regarded as a non-crystalline clay mineral. But the main components are montmorillonite group clay minerals.

In addition to montmorillonite group clay minerals, the following montmorillonite-like three-layer type (2:1 type) clay minerals are also employable in the present invention. That is, pottery stones obtained in Nagasaki Prefecture (Goto Mine), Okayama Prefecture (Mitsuishi Mine) and Nagano Prefecture (Onba Mine, Yonago Mine) of Japan contain pyrophyllite as the main component. These pottery stones are distinguished from montmorillonite clays in that their magnesium content is very low and they scarcely exhibit swelling property, but they resemble montmorillonite in that they are of a laminated structure having a three-layer type (2:1 type) composite layer of silica - alumina - silica as a basic unit.

Talc obtained in Hyogo, Okayama, Hiroshima, Yamaguchi and Nagasaki districts of Japan contains a small amount of aluminum and a specially large amount of magnesium, and in this point it is different from montmorillonite, but it also is of a laminated structure based on a three-layer type (2:1 type) composite layer of silica - alumina - silica.

Among the clays obtained in Kumamoto and Niigata districts of Japan there are found many illites, which are classified minutely into hydromica, glauconite, muscovite, mica, illite, etc. according to their content of iron, fluorine, magnesium, etc. All of these illites have large potassium contents and in this point they are distinguished from montmorillonite, but they are also three-layer type (2:1 type) clay minerals of silica - alumina - silica.

Vermiculite obtained in Brazil, America (Pennsylvania) and India have long been given attention also in Japan as "Hiru-Ishi" and "Hiru-Suna", but it is different from montmorillonite in that its magnesium content is high. However, vermiculite has a laminated structure based on a three-layer type composite layer consisting of silica (tetrahedron) - alumina and magnesia (octahedron) - silica (tetrahedron), and in this point it is similar to montmorillonite.

In addition to the foregoing three-layer type clay minerals, any of which may be used in the invention, artificially prepared three-layer type clay minerals are also employable. For example, taeniolite obtained in South Greenland belongs to illites rich in potassium, fluorine and magnesium, but it is obtainable also by mixing and melting sodium fluoride, lithium fluoride, magnesium oxide and silicon dioxide. Such an artificially prepared taeniolite may be used in the invention.

In the present invention it is preferable that the foregoing three-layer type clay minerals be used as powder having a particle size not larger than 0.15 mm (100 mesh). The foregoing bentonite is convenient because usually the greater part thereof (50-95%) is occupied by fine particles of diameters smaller than 74 $\mu$m (200 mesh) and it is rich in crude clay components (0.2-2 $\mu$m) and fine clay components (below 0.2 $\mu$m). Talc is commercially available as 150-270 mesh (0.1-0.05 mm) powder for cosmetic use. In case 30 to 100 mesh (0.59-0.15 mm) granules of acid clay, active white earth, Fuller's earth, pyrophyllite, illite and vermiculite are used, there is the tendency that the mechanical strength of the resulting gel becomes non-uniform. Therefore, it is recommended to use those granules after pulverization to not less than 100 mesh, preferably not less than 150 mesh.

In the present invention, powder of the foregoing clay minerals is added and dispersed into the foregoing aqueous polyvinyl alcohol solution, or a suspension of clay minerals is prepared in advance and it is mixed into the foregoing aqueous polyvinyl alcohol solution. Furthermore, polyvinyl alcohol may be added and dissolved in the suspension of clay minerals.

In any case, the concentration ratio (weight ratio) of polyvinyl alcohol to clay minerals in the resulting aqueous suspension of polyvinyl alcohol and clay minerals should be not less than 1/5 as previously noted, that is, the concentration of clay minerals suspended should be not more than 5 times the concentration of polyvinyl alcohol. If larger amounts of clay minerals are used, as previously noted, the resulting gel tends to exhibit a lower mechanical strength, and this tendency is particularly remarkable in the case of using 10 times or more amounts of clay minerals with respect to polyvinyl alcohol. Three-layer type clay minerals, as long as they are not used in such excess amounts, contribute to both high water content and high mechanical strength of gels obtained in the present invention. Attaining such high water content and high mechanical strength has heretofore been considered to be a difficult problem incompatible with each other. In this point the clay minerals used in the present invention exhibit a unique effect which has heretofore not been anticipated. The contribution of those clay minerals is particularly remarkable when using three-layer type clay minerals in amounts of one-fifth to one-fifteenth of polyvinyl alcohol (that is, at concentration ratios of polyvinyl alcohol to clay minerals in the range of from 5/1 to 15/1).

The aqueous suspension thus obtained is not always neutral. For example, in case bentonite is used, the suspension often exhibits a pH value of 8 to 10, and in the case of an acid clay, the pH is 3 to 6. On the other hand, for the growth and action of live microorganisms to be immobilized in the present invention, there exist suitable pH ranges, for example: Escharichia coli 7-7.5, Saccharomyces cerevisiae 5-7, Ashbya gossypii 6.8-7.0, Bacillus subtilis 4.8-6.8, Acetobacter acetosum 3.5-6.5, Trametas sanguinea 2-6. Therefore, in order to make the pH value of the foregoing aqueous suspension coincident with a suitable pH value of the live microorganism to be immobilized, it is recommended to use a pH adjustor, typical of which are hydrochloric acid, sulfuric acid, nitric acid, citric acid, sodium hydroxide and potassium hydroxide.

In the present invention, an aqueous polyvinyl alcohol solution or a mixed aqueous suspension of a polyvinyl alcohol and three-layer type (2:1 type) clay minerals is sterilized prior to incorporation therein of a live microorganism. Needless to say, in the present invention, the aqueous suspension containing a polyvinyl alcohol, a live microorganism and if necessary clay minerals may also be prepared by preparing an aqueous suspension of the live microorganism at first, then adding therein the polyvinyl alcohol and if necessary the clay minerals. If the sterilization is made at 100°C for 5 minutes, its purpose may be attained, but in case the aqueous solution or suspension is contaminated by thermostable bacteria, there should be applied sterilization with steam under high pressure at 120°C for 15 minutes to 6 hours. The ultraviolet irradiation method is also employable, but its effectiveness is limited to the irradiated surface, so its combination with the aforesaid heat-sterilization method is desirable. Anyhow, the application of these treatments will not cause a change in quality of the materials used in the present invention, and the invention will not be impeded at all in its working. The sterilized aqueous solution or suspension is then mixed with a live microorganism to be immobilized.

In the immobilizing step, as previously noted, the present invention uses no acid, alkali, radiation, radical forming agent, organic solvent, or reagent, so the live microorganism immobilized will never be damaged. Consequently, all of thermostable and non-thermostable live microorganisms can be immobilized regardless of whether or not they resist organic reagents, acids, alkalies and radiation. Typical examples of such microorganisms include moulds such as Aspergillus and Phizopus, and bacteria such as Pseudomonas, Acetobacter, Streptomyces and Escherichia, and yeasts such as Saccharomyces and Candida.

As live microorganisms there may be used any of freeze-dried and preserved live microorganisms, culture solutions of live microorganisms, and suspensions of live microorganisms obtained by centrifugal concentration from the culture solutions.

As to the incorporating operation for live microorganisms, it is convenient to perform it in a sterile room at room temperature, but in the case of immobilizing thermostable,

0060052

live microorganisms, it is rather preferable, from the standpoint of prevention of saprophytes contamination, to perform such operation at temperatures above room temperature according to their respective thermostabilities. At low temperatures, the viscosity of the aqueous suspension increases and the mixing and dispersion of the live microorganism are relatively slow, but if attention is paid to this point, the operation in question may be carried out at about 0-15°C.

As to the amount (on a dry matter basis) of a live microorganism to be added, it is preferable, from the standpoint of immobilizing nearly the total amount of the live microorganism, that it be not more than seven times the total amount of polyvinyl alcohol or that of polyvinyl alcohol and clay minerals in the aqueous suspension. If this condition is followed, 96% to 98% of the live microorganism can be sure to be entrapped (embedded, immobilized) after going through dehydration (gelling) step as will be described later. When the gel obtained after going through freeze-molding and dehydration steps was observed through a scanning electron microscope, the interior of the gel proved to be porous, and a solid phase (polyvinyl alcohol insoluble in cold water and warm water, or the polyvinyl alcohol and clay) and a liquid phase (water phase) commingled each other and assumed the state of a labyrinth of waterways. These waterways, which are about 1/2 to 100 μm wide, meander continuously and complicatedly. Therefore, once a live microorganism (1-10 μm) is entrapped even in a small quantity into these continuously meandering waterways, the live microorganism can be propagated as a matter of course by having a culture solution (nutrient medium) penetrate therein.

The gel obtained in the present invention has such an advantage in its internal structure, so the amount (initial immobilization amount) of a live microorganism to be added is not limited; for example, it can be selected in the range of 1/1000 to 7 times by weight based on the amount of polyvinyl alcohol or the total amount of polyvinyl alcohol and clay minerals.

According to the method of the present invention, the mixed aqueous suspension of a polyvinyl alcohol and a live microorganism or the mixed aqueous suspension of a polyvinyl alcohol, clay minerals and a live microorganism, which has been prepared in the hereinabove described manner, is then poured into a desired shape of a pre-sterilized vessel or mold and then freeze-molded while paying attention to avoid incorporation of saprophytes and to avoid direct irradiation of a germicidal lamp (ultraviolet ray). In this case, using a projections-disposed plate as such vessel or mold is one of preferred modes as will be described later.

As a refrigerant there may be used, for example, a freezing mixture such as common salt - ice (23:77) (-21°C) or calcium chloride - ice (30:70) (-55°C), or dry ice - methyl alcohol (-72°C), or liquid nitrogen (-196°C), thereby cooling the aqueous suspension to a temperature lower than -6°C. If cooling is insufficient, the gel obtained after going through a dehydration step as will be described later will be inferior in its mechanical strength. The use of liquid helium would permit cooling down to -269°C, but it is not only uneconomical but also is not advantageous to the quality of the resultant gel. Practically, it is recommended to use a Freon refrigerator for cooling to, for example, not higher than −20°C and further not higher than −35°C. It is not preferable for many live microorganisms to be exposed to temperatures near −20° to −30°C for a long time, so it is rather preferable to cool them rapidly down to below −30°C, for example, −35° to −80°C. Freeze-molding at such a low temperature will contribute to enhancing the mechanical strength of the live microorganism supporting gel, and it is preferable to the freeze-molding at a temperature between −20° and −6°C. Even if the freeze-molding step is omitted and the dehydration as will be described later is applied directly, gel can be formed, but in this case the aqueous suspension must be spread plane-wise, so it is very difficult to mold it into various shapes, including spherical, cylindrical, columnar, lumpy, annular and spiral shapes. From the standpoint of working efficiency, therefore, such a direct application of dehydration is usually limited to the manufacture of the foregoing plate-like or filmy gel. In this case, moreover, even for obtaining a gel about 10 mm in thickness, a long period is needed for drying the aqueous suspension. Besides, it is difficult to dehydrate uniformly up to a deep portion of gel, and the mechanical strength of the resultant gel is far inferior to that of the gel of the present invention.

In the freeze-molding according to the method of the present invention, the aqueous suspension is solidified (frozen) and molded within a desired shape of a mold, then the upper cover or lower cover (or both covers) of the mold is removed and the molded article is subjected to freeze-dehydration while keeping its shape. Adoption of the freeze-dehydration method is advantageous in that the molded article can be dehydrated to its deep portion nearly uniformly and that rapidly. In the present invention, therefore, the freeze-molding step is very important. Besides, the execution of freeze-molding at low temperatures brings about a quite unexpected effect such that it contributes to the improvement of mechanical strength of the resultant polyvinyl alcohol gel or polyvinyl alcohol - clay minerals gel. Also in this point, the freeze-molding in the present invention is of great significance. As to the cooling rate in the freeze-molding operation, with the influence on the foregoing live microorganisms taken into account, a slow cooling at a rate of 0.1 to 7°C/min may be applied for cooling to -10°C or so, but thereafter a rapid cooling at a rate of 7 to 1,000°C/min is preferred.

The gel which has immobilized a live microorganism is often desired from the aspect of its use to have the shape of a thin-layer sheet (film). The use of a projections-disposed plate as the vessel or mold to obtain a perforated thin-layer sheet (net-like molding) will afford remarkable effects in point of manufacture and in the properties of the resultant gel. Even if the aqueous suspension is applied onto a plate having no projections on the surface thereof followed by the application of freeze-molding and subsequent vacuum-dehydration as will be described later, gel is formed, but a highly dehydrated hard film having a water content of not higher than 20 wt.% is apt to be formed because the freezing and dehydration of such a thin-layer sheet (film) proceeds relatively rapidly. This excessively dehydrated thin-layer plate (film) is not satisfactory as a live microorganism immobilizing carrier in point of elasticity and water absorbing property. Moreover, because it assumes the shape of a mere thin-layer sheet (film), its charging, for example, into a reaction column would cause blocking-up of the same column so is not desirable.

Furthermore, a thin-layer sheet (film) having a flat surface when dehydrated to excess contracts about 5% to 20% in the planar direction, so it is difficult to obtain a uniform and smooth dehydrated surface, thus resulting in that there occurs inconvenience in the subsequent shaping operation, including cutting operation. These drawbacks can be overcome by ensuring a residual water content of not less than 20 wt.% (20-92 wt.%) without subjecting the thin-layer sheet (film) to excessive dehydration. However, as previously noted, because the dehydration of a thin-layer sheet (film) proceeds relatively rapidly and because damages to gel (deterioration of water absorbing property and of elasticity, planar shrinkage) caused by excessive dehydration are conspicuous particularly in the case of a thin-layer sheet (film), it is desired to think out a countermeasure. Moreover, with a view to ensuring gas-liquid passage in the reaction column, it is desired to produce a perforated plate-like (reticulately molded) gel (live microorganism embedded gel) different from a mere thin-layer sheet (film).

All of these problems can be solved by the freeze-molding and dehydration system using a projections-disposed plate. That is, according to the present invention, even if the dehydration (drying) should be done to excess, there is obtained a uniform and smooth dehydrated gel without causing shrinkage in the planar direction, and a gel (live microorganism embedded carrier) superior in all of wet strength, wet elasticity and water absorbing property is obtainable; in addition, it is possible to obtain a reticulate (perforated plate-like) molded gel having many flowing paths of gases and liquids.

As the projections-disposed plate there may be used a flat plate or a curved (corrugated) plate having 900 to 500,000 projections per square meter. If the density of projections is too low, an excessive dehydration would cause gel to crack or shrink. Therefore, the distance between projections should be not more than 5 cm, preferably not more than 2.5 cm, and there should be not less than 900, preferably not less than 2,500, projections per square meter. If the projection density is too high, the resultant molded gel will be deteriorated in its mechanical strength.

As to the thickness of projections, if it is too small, there is fear that gel will be torn off by the projections, and also in consideration of the durability for washing and repetitive use of the projections-disposed plate after its use, it is recommended that the thickness of projections be not less than 0.1 mm, preferably not less than 1 mm. The height of projections is decided according to the thickness of a desired, molded gel. For example, it may range from 0.01 to 5 mm.

Those projections and projections-disposed plate may be formed of any material typical of which are polyethylene, polypropylene, Teflon, steel, aluminum and cast iron. But, hard glass, ordinary glass and pottery are not desirable because

0060052

they are often broken in the course of rapid freezing (cooling) as will be described later.

The foregoing mixed aqueous polyvinyl alcohol suspension is poured or applied with a spatula or the like onto the surface of the projections-disposed plate. The application thickness should be 0.01 to 5 mm, preferably 0.1 to 3 mm, and it may reach the same height as the projections. In case the mixed aqueous suspension of a polyvinyl alcohol and a live microorganism or the mixed aqueous suspension of a polyvinyl alcohol, clay minerals and a live microorganism is subjected to freeze-molding and dehydration after its application onto the projections-disposed plate according to the present invention, their shrink percentages in the direction of the thickness are 3-8% and 2-6%, respectively, and openings corresponding to the density of the projections are formed in the dehydrated gel. It is to be noted, however, that if those aqueous suspensions are applied more thickly than the height of the projections, the object of forming openings (molding a perforated plate-like gel) is often unattainable.

Furthermore, in case the aqueous suspension is applied beyond 1.3 times the height of the projections and the dehydration is made to the extreme degree, not only the object of forming openings is unattainable, but also the dehydrated gel often shrinks in the planar direction and its water absorbing property is deteriorated; as a result, the effect of the projections-disposed plate used in the present invention is almost lost. Therefore, even in the case where a perforated thin-layer sheet (film) is not aimed at, it is recommended that the application thickness be smaller than 1.3 times, preferably not larger than 1.2 times, the height of the projections. If the projections-disposed plate is used, gel (freeze-molded gel) is fixed at a large number of projected points, so the shrinkage of gel in the planar direction (shrinkage by dehydration) is prevented. Rather, the gel is dehydrated in such a state that a tensile force is exerted between the gel fixing points, and this is presumed to induce the remarkable effects.

In the present invention, the mixed aqueous suspension of a polyvinyl alcohol and a live microorganism or the mixed aqueous suspension of a polyvinyl alcohol, clay minerals and a live microorganism is subjected to vacuum-dehydration after confirming that it was frozen. In this case, the frozen molding is taken out of a refrigerating room,,then transferred into a vacuum-dehydration room and immediately dehydrated by means of suction whereby the sample is cooled along with removal (sublimation) of water, so the frozen molding will never thaw even without special cooling from the exterior. Heating may be applied to the extent that the frozen molding does not thaw, whereby dehydration can be accelerated. That is, as long as the frozen molding is not thawed, the dehydration temperature is not specially limited and it will not have a special influence upon the quality of gel.

In the present invention, regardless of the concentration of polyvinyl alcohol and that of clay minerals, the dehydration treatment (vacuum-dehydration) is applied to the frozen molding. In this case, dehydration percentages (percentage reduction in weight of the frozen body) of not less than 5 wt.%, usually

0060052

not less than 15 wt.%, for example, 5 to 90 wt.% are adopted. Dehydration may be done up to an almost completely dehydrated state, for example, up to a water content of about 1-10 wt.%. Also, the water content may be about 10-92 wt.%. As the dehydration proceeds, the strength of gel is enhanced and therefore it is recommended to select the dehydration amount according to the desired strength of gel.

The dehydration step (freeze-dehydration) cannot be omitted. Without going through this step, the highly elastic and mechanically strong gel of a high water content of the present invention would not be obtained and hence the live microorganism-immobilized gel obtained would be very weak. (If the frozen molding is thawed and then dehydrated under reduced pressure without retaining the frozen state of the mixed aqueous suspension of a polyvinyl alcohol and a live microorganism, the thawed solution will bubble vigorously to the extent that the operation cannot be continued. Besides, even if the dehydration is made over an extended period, there will merely be produced a whitely turbid, less elastic gel.)

The degree of vacuum in the vacuum-dehydration step of the present invention is not limited so far as the frozen water can be dehydrated. Usually, less than 10 mmHg, preferably less than 1 mmHg, more preferably less than 0.1 mmHg, can be adopted.

In the present invention, the freeze-molded and dehydrated article of the mixed aqueous suspension of a polyvinyl alcohol and a live microorganism or the mixed aqueous suspension of a polyvinyl alcohol, clay minerals and a live microorganism is then allowed to stand at room temperature to thaw it whereby there is obtained a microorganism immobilized gel rich in elasticity. In this case, in addition to a slow thawing at a rate of 1-3°C/min, a rapid thawing at a rate of 3-1000°C/min may be adopted as the case may be in consideration of the thermostability of the live microorganism used. Anyhow, at temperatures above 60°C a hard film is rapidly formed on the surface of gel and therefore, regardless of the thermostability of the live microorganism used, it is desirable to perform the thawing operation at a temperature of 40° to 50°C or lower. In water, the gel absorbs water and its water content reaches 50 to 95 wt.% (on a wet body basis), but the gel is still a strong elastic body suitable for the growth and action of the microorganism embedded therein. As is apparent from the foregoing result of observation through the scanning electron microscope and from the above-mentioned water content (50-95 wt.%), the greater part of the interior of the gel is occupied by holes (aqueous phase). This water content of the gel, though not so high as that of devil's tongue jelly (wet polysaccharide gel having a water content of about 97 wt.%), is similar to the water content (70-90 wt.%) of living body cells and the tissue of living bodies such as human beings and animals. Besides, in point of strength and elasticity, this gel by far surpasses polysaccharide gels such as devil's tongue jelly, agar, alginic acid, carrageenan, guar gum, locust been gum, agarose and tragacanth gum, and it resembles the tissues of living bodies such as human beings and animals.

In contact with water the gel of the present invention absorbs a large amount of water, but it exhibits an elasticity, and when it is squeezed firmly, it is deformed for a moment, but immediately reverts to its original shape, and thus it does not get out of shape. In this case, the water contained therein scarcely oozes out. For example, when a compressive stress of 2 $kg/cm^2$ is applied to gel with a water content of 90 wt.%, the amount of water oozed out (flowed out) is only 1% to 2%. Also, the tensile strength is as high as 4 $kg/cm^2$, and thus the gel, though its water content is very high, proves to be a very superior elastic body.

Attaining both high water content and high mechanical strength has heretofore been considered to be a difficult problem incompatible with each other in the development of medical high polymers and selectively permeable membranes, but the gel of the present invention, as mentioned above, has a high water content and a high strength. It is quite different from conventional films obtained by air-drying an aqueous polyvinyl alcohol solution or from weak gels formed when an aqueous polyvinyl alcohol solution is merely allowed to stand at 0-30°C as previously noted or when it is merely frozen and thawed.

Thus, as is apparent from the fact that the gel of the present invention firmly retains a large amount of water, the apparent specific gravity of this gel is about the same as that of water. It barely precipitates in water. In case carbon dioxide gas is evolved along with the growth and action of the live microorganism embedded in the gel, the existence of this gas in the gel, even if its quantity is small, keeps the apparent specific gravity of the gel not higher than 1.0, and the gel rather floats in water.

In case the gel of the present invention is obtained using a projections-disposed plate, the resultant molded article assumes the shape of a perforated plate. Consequently, the opening percentage is increased, and although a decrease in apparent mechanical strength as compared with the intrinsic strength of the gel is unavoidable, the apparent tensile strength reaches as high as 2-3 $kg/cm^2$ for example in the case of a net-like molded article having an opening percentage of 50%. Thus, such a perforated molding can be put to practical use sufficiently as a live microorganism immobilizing carrier (net).

The gel of the present invention has no stickiness. Even when 10 g. of the gel (molded article) of the present invention is immersed in 50 ml. of water and stirred for 10 days, the phenomenon of mutual adhesion or getting out of shape is not recognized at all. When the gel was immersed in tap water for a period of one year, it did not dissolve and its elasticity and strength did not change. (This is in striking contrast to the case of devil's tongue jelly which when immersed in tap water for several days gets out of shape noticeably.)

In the present invention, a single polyvinyl alcohol component or the combination of a polyvinyl alcohol and three-layer type (2:1 type) clay minerals is used as a gel material (gelling component). In this case, three-layer type (2:1 type) clay minerals contribute to the improvement in mechanical strength and water absorption of gel and further contribute to diminishing the planar shrinkage of gel when dehydrated (dried) to the extreme degree. Therefore, by using three-layer (2:1 type) clay minerals, there can be produced a gel having a fairly high strength even from an aqueous polyvinyl alcohol solution with a relatively low concentration of polyvinyl alcohol.

Zeolite, silica-alumina, synthetic aluminum silicate, polyethylene glycol, casein, gum arabic and asbestos also exhibit effects fairly similar to three-layer type (2:1 type) clay minerals although a detailed mechanism is not clear. Co-existence of inorganic or organic substances having nothing to do with the gelation of polyvinyl alcohol would cause no trouble in the present invention. Such inorganic or organic substances may be present in an amount of, for example, not more than one half of the amount by weight of polyvinyl alcohol. On the other hand, substances which act on polyvinyl alcohol (or modified polyvinyl alcohols such as polyvinyl acetal and polyvinyl butyral) to form a composite gel, and substances which react with polyvinyl alcohol and modifies the latter, even if their co-existent amounts are small, often exert an undesirable influence upon the gelation in the present invention and make it difficult to obtain a gel of high water content superior in mechanical strength. As examples of such substances there may be mentioned the following substances whose interaction with polyvinyl alcohols are already known: colloidal alkali silicate [see U.S. Patent No.2,833,661 (1958)]; colloidal silica [U.S. Patent No.2,833,661 (1958)]; alkaline colloidal silica (Japanese Patent Laid Open No.153779/1979); organic silicon compounds [Saksan Vinyl Jushi, Nikkan Kogyo Shinbun-Sha (1962), p.93]; tetraalkyl silicate (Japanese Patent Publications Nos.30358/1980 and 11311/1980);

boric acid and borax [French Patent No.743942 (1933)]; phenol, naphthol, m-creson, pyrogallol, salicylanilide, disalicylbenzidide, resorcinol and polyamines [Kobunshi Kagaku, 11, (105) 23 (1954)]; kaolin [Nature, 170, 461 (1955)]. The use of these substances should be avoided because they form a composite, inconvenient gel with polyvinyl alcohol according to their amounts of co-existence.

In the present invention, as previously noted, it is essential that the aqueous suspension of a live microorganism be subjected to freeze-molding and dehydration. It has long been well known that, in general, in the case of freeze-dehydrating microorganisms or living body tissues or their suspensions, they suffer more or less damage from freeze-dehydration. As the method for avoiding or remarkably diminishing such a freeze-dehydration damage to living bodies or tissues thereof or to proteins, the addition (co-existence) of small amounts of carboxyl group-containing water-soluble high polymers such as carboxymethyl cellulose or various protectants against freeze-dehydration damage is well known in addition to the foregoing method of rapidly cooling to a low temperature not higher than -30°C. In the present invention, polyvinyl alcohol (gelling component) per se acts as a powerful protectant against freeze-dehydration damage whereby most of live microorganisms usually are protected and their sufficient growth and action are ensured as will be described later, but known freeze-dehydration damage protectants may co-exist.

Substances for diminishing freeze-dehydration damage are generally called protectant, protective substance, additives, additional substance, medium, adjuvant, suspended medium, or stabilizer. As examples of such substances there are known magnesium ion, glycerin, dimethyl sulfoxide, honey, peptone, meat extract, yeast extract, skim milk, serum, albumin, sodium salt and potassium salt of L- or D-glutamic acid, N-acetyl glutamate, D- or L-arginine, DL-2-pyrrolidone-5-carboxylate, polyvinyl pyrrolidone, L-homoalginic acid, D-glucose, D- or L-aspartic acid, ascorbic acid, DL-threonine, D,L-allothreonine, gelatin, mucin, lactose, DL-malic acid, L-cysteine, L-sorbitol, arabitol, pectin, gum arabic, mannose, galactose, L-lysine, D-fructose, dextrin, dextran, sucrose, soluble starch, raffinose, citric acid, acetylglycine, and D-xylitol. Also known are combination uses such as L-glutamate/skim milk (or dextran, soluble starch, polyvinyl pyrrolidone, carboxymethyl cellulose, gelatin, lactose); dextran/ammonium chloride/thiourea/ascorbic acid; skim milk/ascorbic acid (or sucrose), and glucose/serum. If these substances added 0.5% to 2% to the foregoing aqueous polyvinyl alcohol solution, the purpose of their co-existence is fully attained in many cases, but they may be added about 10% by weight.

In case the molded gel immobilizing a live microorganism formed according to the method of the present invention, for example, a perforated thin-layer net 1 mm thick having an opening percentage of 50% or a similar net 2 mm thick having an opening percentage of 70%, is cut into pieces each 10cm by 10cm in size and these cut pieces, or Raschig ring-shaped molded pieces 8mm by 8mm, are added into an aqueous substrate solution (medium) having a temperature and pH value suitable for the growth and action of the immobilized live microorganism, there is observed an activity of about 85-97% as compared with the original (unimmobilized) live microorganism. If the size of such molded gel is further increased, the above relative activity tends to decrease, but in this case, if required, there may be used known substrate diffusion accelerators, namely, surfactants such as sulfuric ester of lauryl alcohol, cetylpyridinium chloride, and cetyltrimethylammonium bromide, the use of which would not deteriorate performances such as mechanical strength and life of the gel.

If the polyvinyl alcohol gel or the composite polyvinyl alcohol - clay gel of the present invention is subjected to a hardening treatment for the polyvinyl alcohol fiber or film, the mechanical strength of the gel is somewhat enhanced. There may be applied known hardening (cross-linking) treatments, for example, using aldehydes, dialdehydes, diisocyanates, phenols, or metallic compounds such as titanium, chromium and zirconium compounds, or borax, acrylonitrile, trimethylolmelamine, epichloro-hydrin, bis-($\beta$-hydroxyethyl)sulfone, polyacrylic acid, dimethylolurea, maleic anhydride. However, when it is taken into account that the gel of the present invention has a strength (resistance to load) and that the aforesaid auxiliary hardening treatment often results in damage to the immobilized live microorganism, it is rather preferable in the present invention to avoid such a hardening treatment.

The following examples are given to further illustrate the invention.

0060052

Example 1

86 g. powder (water content 7 wt.%) of a commercially available polyvinyl alcohol [degree of hydrolysis: 97 mol%, viscosity-average polymerization degree: 1,700, viscosity as a 4% aqueous solution: 26 cP (20°C)] was dissolved in 914 g. of water to prepare a 8.0 wt.% aqueous solution thereof.

44 g. of this aqueous solution wss sterilized with steam under pressure at 120°C for 20 minutes and then allowed to cool in a sterile room. Thereafter, 4 g. of a suspension (phosphoric acid buffer solution, pH 7) containing 0.8 g. of Saccharomyces cerevisiae was added and stirring was made for 7 minutes to give an aqueous suspension containing 7.3 wt.% of the polyvinyl alcohol. 40 g. of the aqueous suspension, in a sterile room, was poured into a Raschig ring (outside dia. 8mm, inside dia. 4mm, length 8mm) forming mold (for 130 pieces) and cooled (freeze-molded) at -33°C for 0.5 hour. Then, the molded articles were taken out and vacuum-dehydrated for 6 hours at 0.1 mmHg. After thawing, there was obtained 23 g. (water content 84 wt.%, dehydration percentage ≡ loss in weight of the freeze-molded body = 43 wt.%) of a molded gel (yeast-containing Raschig ring). This molded gel was immersed in 40 ml. of a pre-sterilized 0.9% saline solution for 6 hours as a result, the gel absorbed water and increased in weight to 27 g. (water content 87 wt.%). The aforesaid yeast was not detected from this immersion solution, and from this fact it is seen that almost all the quantity of the yeast was embedded (entrapped) in the Raschig rings.

27 g. of the Raschig rings were charged irregularly into a glass column 3cm dia. by 10cm high, then an ethyl alcohol preparing aqueous substrate solution (glucose 10 wt.%, magnesium sulfate heptahydrate 60 ppm, pH 6, 32°C) was introduced from the bottom of the column at a rate of 25 ml/h. After 12 hours, the concentration of ethyl alcohol in the effluent reached 4 wt.% (77% of theory). After this operation was continued for 12 days, the concentration of ethyl alcohol in the effluent proved to still 4 wt.%.

Example 2

84 g. powder (water content 5 wt.%) of a commercially available polyvinyl alcohol [degree of hydrolysis: 98.4 mol%, viscosity-average polymerization degree: 1,800, viscosity as a 4% aqueous solution: 29.5 cP (20°C)] was dissolved in 916 g. of water to prepare a 8.0 wt.% aqueous solution (pH 6.9) thereof.

18 g. of this aqueous solution was sterilized with steam under pressure at 120°C for 20 minutes and then allowed to cool in a sterile room. Thereafter, 2 g. of a suspension (phosphoric acid buffer solution, pH 7) containing 0.4 g. of Saccharomyces cerevisiae was added and stirring was made for 7 minutes to give an aqueous suspension containing 7.2 wt.% of the polyvinyl alcohol.

18 g. of the aqueous suspension, in a sterile room, was poured into a polyethylene vessel (bottom 6cm x 6cm), cooled (freeze-molded) at -53°C for 0.6 hour and then vacuum-dehydrated for 5 hours. After thawing, there was obtained 10.5 g. (water content 84 wt.%, dehydration percentage 42 wt.%) of a white opaque gel. The gel was immersed in 30 ml. of a pre-sterilized 0.9% saline solution for 6 hours; as a result, the gel absorbed water and increased in weight to 12 g. (water content 86 wt.%). The above-mentioned yeast was not detected from this immersion solution.

The gel was then cut into many small pieces (8mm x 8mm x 4mm) and the cut pieces were washed with 40 ml. of a pre-sterilized 0.9% saline solution. This washing when observed with an optical microscope proved to contain a small quantity of the aforementioned yeast, but as a result of quantitative determination made on the basis of its turbidity, it became clear that at least 98% of the initial yeast was firmly embedded in the gel (small pieces).

40 ml. of an alcohol preparing aqueous substrate solution comprising 5 wt.% of glucose and 60 ppm of magnesium sulfate heptahydrate was charged into a Sakaguchi flask (500 ml.), sterilized at 120°C for 20 minutes and then allowed to cool in a sterile room. Thereafter 12 g. of the aforesaid cut gel (immobilizing yeast) was added and the flask, after putting a burnt cotton plug, was shaken in a thermostatic room held at 30-33°C. Ajter 24 hours, 1.9 wt.% of ethyl alcohol was detected from the aqueous substrate solution, while the yeast was not recognized therein.

On the other hand, 40 ml. of the above aqueous substrate solution was added into 12 g. of a suspension containing 0.4 g. of Saccharomyces cerevisiae of the same strain as the above yeast, followed by shaking in the same manner. As a result, after 24 hours, the ethyl alcohol concentration of the suspension reached 2.2 wt.% (86% of theory).

Thus, it is apparent that the yeast embedded in the gel of the present invention was kept immobilized in the gel throughout the fermentation operation for the alcohol and that its glycolysis activity (ethyl alcohol producing ability) reached 90% of the original yeast (unimmobilized yeast).

Comparative Example 1

60 g. of an 8.0 wt.% aqueous solution of the polyvinyl alcohol described in Example 2 was sterilized with steam under pressure at 120°C for 20 minutes and then allowed to cool. Thereafter, 7 g. of a suspension (phosphoric acid buffer solution) containing 1.4 g. of Saccharomyces cerevisiae of the same strain as that used in Example 2 was poured into the above aqueous solution and stirring was made for 7 minutes. 60 g. of the resulting aqueous suspension was poured into a vessel having a bottom 10cm by 10cm and allowed to stand for 2 days to give 14.5 g. (water content 62 wt.%) of a weak, sticky film like a wet cellophane paper. This film (about 1.5 mm thick) was immersed in a pre-sterilized 0.9% saline solution; as a result, after 4 hours, the weight of the film increased to 17.2 g. (water content 67 wt.%). A fairly large quantity of the yeast was recognized in this immersion solution. As a result of turbidimetric analysis, it became clear that 15% of the yeast was transferred into the immersion solution without being embedded in the polyvinyl alcohol film. The film was then cut into many pieces (8mm x 8mm x 1.5mm) and the cut pieces were washed with 84 ml. of a pre-sterilized 0.9% saline solution (about 1 wt.% of the yeast based on its initial quantity exuded into the washing).

40 ml. of the aqueous substrate solution described in Example 2 was charged into a Sakaguchi flask (500 ml.), sterilized at 120°C for 15 minutes and then allowed to cool in a sterile room. Then, 17 g. of the above cut pieces of the film were added and the flask, after putting a burnt cotton plug, was shaken in a thermostatic room at 30-33°C. After 24 hours, the concentration of ethyl alcohol was only 0.4 wt.% (15% of theory), and a small quantity of polyvinyl alcohol proved to have dissolved out.

Thus, in the case of following a conventional method using polyvinyl alcohol, not only the loss of yeast in its immobilization step is more conspicuous than in Example 2, but also the glycolysis (ethyl alcohol formation) of the immobilized yeast is very slow. Besides, the resulting film is weak.

Comparative Example 2

The procedure of Example 2 was repeated except that the polyvinyl alcohol used therein was substituted by a commercially available polyvinyl alcohol having a degree of hydrolysis of 93 mol%, a viscosity-average polymerization degree of 1,700 and a viscosity as a 4% aqueous solution of 30 cP (20°C). There was obtained 10 g. of a freeze-molded and dehydrated article (water content 83 wt.%, dehydration percentage 44 wt.%), which after thawing weakened even at 5°C. In addition to a small quantity of gel layer, a large quantity of a concentrated aqueous polyvinyl alcohol solution was separated. At the same time, this separated liquid phase proved to contain a large quantity of the yeast exuded.


Comparative Example 3

The procedure of Example 2 was repeated except that in place of the polyvinyl alcohol used therein there was employed 18 g. of an 18 wt.% aqueous solution of a commercially available polyvinyl alcohol having a degree of hydrolysis of 99.2 mol%, a viscosity-average polymerization degree of 500 and a viscosity as a 4% aqueous solution of 5.6 cP (20°C). As a result, there merely was obtained 10.5 g. of an agar-like weak gel (water content 84 wt.%, dehydration percentage 42 wt.%) which exhibited little elasticity.


Comparative Example 4

The concentration of the same aqueous polyvinyl alcohol solution having a polymerization degree of 500 was increased to 30 wt.%, and 18 g. of this aqueous solution was treated in the same manner to give 10.4 g. of gel (water content 84 wt.%, dehydration percentage 42 wt.%). In water, the gel softened noticeably and got out of shape; at the same time, the yeast flowed out into the water.


Comparative Example 5

65 g. powder (water content 8 wt.%) of a commercially available polyvinyl alcohol [degree of hydrolysis: 99.4 mol%, viscosity-average polymerization degree: 2,600, viscosity as a 4% aqueous solution: 66 cP (20°C)] was dissolved in 93.5 g. of water to prepare a 6 wt.% aqueous solution thereof. 34 g. of the aqueous solution was sterilized with steam under pressure and allowed to cool, then 3 g. of the same aqueous suspension as that used in Example 1 was added. After freeze-molding at −70°C for 0.5 hour and standing for 2 hours at room temperature, there was obtained a soft gel (37 g., water content 94 wt.%, dehydration percentage 0%). The gel was not elastic at all and got out shape when immersed in water overnight. The water layer became turbid and a large quantity of the yeast flowed out into the water. The soft gel before immersion in water was easily ruptured by a tensile stress of only 100 g/cm$^2$.

Thus, it is apparent that even if an aqueous polyvinyl alcohol solution is subjected to freeze-molding, unless it is subjected to subsequent freeze-dehydration, there merely is obtained a weak gel which is inferior in water resisting property to the gel of the present invention and which is not good for practical use while immobilizing yeast.

Comparative Example 6

After the freeze-molding applied in Comparative Example 5, 34 g. of the molded article was thawed at room temperature and then subjected to dehydration under reduced pressure in a vacuum dehydrator, but the thawed solution bubbled so vigorously that the operation was compelled to be distontinued. Then, to avoid bubbling, 1/10 (3.7 g.) of the above thawed solution of the molded article was withdrawn, applied to the bottom of a polyethylene beaker (100 ml.) and subjected to dehydration under reduced pressure. As a result, 1 g. of gel (water content 74 wt.%, dehydration percentage 72 wt.%) was formed on the bottom of the beaker, but this gel was not so elastic and was immediately reptured by a tensile stress of 600 g/cm$^2$.

On the other hand, the gel (yeast-containing gel) obtained in Example 2 according to the method of the present invention was not ruptured at a tensile stress up to 4 kg/cm$^2$, and when a compressive stress of 2 kg/cm$^2$ was applied to the gel and then relieved, the original shape of the gel was restored almost completely.

Thus, it is seen that even if an aqueous polyvinyl alcohol solution is subjected to freeze-molding, unless it is subjected to subsequent dehydration without thawing (while retaining the frozen state), the gel (yeast-containing gel) of the present invention having a high water content and superior mechanical strength rich in elasticity is not obtained.

The elasticity and strength of gel (microorganism-containing gel) are important physical properties not only from the standpoint of expansion of the intra-gel cell (tensile stress exerted on the gelling component near a growing portion and a compressive stress exerted on the gelling component interposed between different growing portions) caused by the growth of the microorganism embedded in the gel. That is, a carrier gel for immobilizing a live microorganism is required to have both an elasticity sufficient to allow a vigorous growing of the microorganism and a strength high enough to resist the expansion of the cell. The gel obtained in this comparative example, as mentioned in Comparative Example 5, is still insufficient in this point as compared with the gels obtained in Examples 1 and 2.

Example 3

86 g. powder (water content 7 wt.%) of a commercially available polyvinyl alcohol [degree of hydrolysis: 97 mol%, viscosity-average polymerization degree: 1,700, viscosity as a 4% aqueous solution: 26 cP (20°C)] was dissolved in 914 g. of water to prepare an 8.0 wt.% aqueous solution thereof (pH 6.8). 170 g. of this aqueous solution was sterilized with steam under pressure at 120°C for 20 minutes and allowed to cool in a sterile room. Then, 20 g. of a suspension (phosphoric acid buffer solution) containing 4 g. of Erwinia herbicola) was added and stirring was made for 7 minutes to obtain an aqueous suspension containing 7 wt.% polyvinyl alcohol.

190 g. of the aqueous suspension was poured in a sterile room into a Raschig ring (8mm x 8mm) forming mold (for 630 pieces) and cooled (freeze-molded) at -53°C for 0.5 hour, then the molded articles were taken out and vacuum-dehydrated for 6 hours. After thawing, there was obtained 133 g. of gel (water content 86 wt.%, dehydration percentage 30 wt.%). When this molded gel was immersed in 100 ml. of a pre-sterilized 0.9% saline solution for 6 hours, it absorbed water and increased in weight to 139 g. (water content 87 wt.%). The aforementioned bacterium was not detected from this immersion solution and therefore it is seen that almost all quantity of the bacterium was embedded in the Raschig rings.

139 g. of the above Raschig rings were charged irregularly into an acrylic resin column 3 cm in diameter and 60 cm height, then a tyrosine preparing aqueous substrate solution (phenol 0.1%, sodium nitrite 0.2%, ammonium acetate 5%, sodium pyruvate 3%, pyridoxal phosphate 100 ppm, ethylenediamine tetraacetate 300 ppm, pH 8, 30°C) which had been sterilized at 120°C for 20 minutes was introduced from the bottom of the column at a rate of 150 ml/h. After 10 hours, the concentration of $\beta$-tyrosine[$\beta$-(p-hydroxyphenyl)alanine] in the effluent reached 600 ppm (30 mol% yield).

Example 4

85 g. powder (water content 6 wt.%) of a commercially available polyvinyl alcohol [degree of hydrolysis: 97 mol%, viscosity-average polymerization degree: 2,200, viscosity as a 4% aqueous solution: 54 cP (20°C)] was dissolved in 915 g. of water to prepare an 8.0 wt.% aqueous solution (pH 6.9).

380 g. of this aqueous solution was sterilized with steam under pressure at 120°C for 20 minutes and then allowed to cool in a sterile room. Thereafter, 20 ml. of a suspension [tris(hydroxymethyl)aminomethane buffer solution, pH 8.0] containing 4 g. of Lactobacillus brevis was added and stirring was made for 7 minutes to prepare an aqueous suspension containing 7.6 wt.% polyvinyl alcohol.

200 g. of the aqueous suspension was poured in a sterile room into a Raschig ring (8mm x 8mm) forming mold (for 665 pieces) and cooled (freeze-molded) at -45°C for 0.5 hour, then the upper cover of the mold was removed and a 6 hours' vacuum-dehydration was applied to the lower cover supporting the moldings. After thawing, there was obtained 130 g. of gel (water content 87 wt.%, dehydration percentage 35%). When this molded gel was immersed in 150 ml. of a pre-sterilized 0.9% saline solution for 6 hours, it absorbed water and increased in weight to 139 g. (water content 88 wt.%). The aforementioned bacterium was not recognized in this immersion solution and therefore it is seen that almost all quantity of the bacterium was embedded in the Raschig rings.

139 g. of the molded gel was charged irregularly into the column described in Example 3, and a fructose preparing aqueous substrate solution (glucose 5 wt.%, manganese sulfate tetrahydrate 0.01 mol/$\ell$ , 62°C, pH 6.3) which had been sterilized at 120°C for 20 minutes was introduced from the bottom of the column at a rate of 120 ml/h. After 35 hours, the pH of the effluent was 6.7 and the fructose concentration thereof was 2.0 wt.% (40 mol% of theory).

Example 5

In Example 4, Streptomyces phaeochromogenes was used in place of Lactobacillus brevis in an amount of 1/200 of the amount used in Example 4, that is, 20 ml. of a suspension (culture medium) containing 0.002 g. of the bacterium was used.

200 g. of a mixed aqueous suspension of the polyvinyl alcohol and the bacterium was freeze-molded and dehydrated in the same manner to give 128 g. of gel (water content 87 wt.%, dehydration percentage 36 wt.%). When this molded gel was immersed in 150 ml. of a pre-sterilized 0.9% saline solution for 6 hours, it absorbed water and increased in weight to 139 g. (water content 88 wt.%).

The gel (137 g.) was charged irregularly in the column described in Example 3, and a culture solution (xylose 1%, peptone 1%, meat extract 1%, yeast extract 0.3%, common salt 0.5%, magnesium sulfate heptahydrate 0.06%, 30°C) which had been sterilized at 120°C for 15 minutes was introduced from the bottom of the column at a rate of 120 ml/h over a period of 48 hours. Before and after this operation for introducing the culture solution (medium), part of the packing (Raschig rings) was sampled and observed with a scanning microscope. As a result, before the said operation, the bacterium was little recognized in the gel, but after the operation, a bacterial colony (about 50um, the number of bacteria: about 300,000) was recognized in many portions in the gel.

Then, a fructose preparing solution (glucose 5%, magnesium sulfate 0.01M, pH 8.3, 65°C) was introduced from the bottom of the column at a rate of 90 ml/h. After 28 hours, the fructose concentration of the effluent reached 2 wt.% (40 mol% of theory) and the pH thereof was 6.3.

When the above-mentioned culture (growth) operation was omitted and the substrate solution was introduced immediately over a 28 hour period, the fructose concentration of the effluent proved to be only 0.02 wt.%.

Thus, in the present invention a live microorganism can be immobilized and therefore, as a matter of course, it is possible to grow it in gel.

Example 6

84 g. powder (water content 5 wt.%) of a commercially available polyvinyl alcohol [degree of hydrolysis: 97 mol%, viscosity-average polymerization degree: 1,800, viscosity as a 4% aqueous solution: 29.5 cP (20°C)] was dissolved in 916 g. of water to prepare an 8.0 wt.% aqueous solution. (pH 6.9).

100 g. of a commercially available bentonite (powder for reagent, water content 12 wt.%) was dispersed in 1,500 g. of water to prepare a 5.5 wt.% bentonite suspension (pH 10.4), to which was added 4 ml. of 6N sulfuric acid to adjust the pH of the suspension to 7.0.

60 g. of the above aqueous polyvinyl alcohol solution and 100 g. of the above bentonite suspension were mixed, then sterilized with steam under pressure at 120°C for 6 hours and allowed to cool in a sterile room. Thereafter, 20 g. of a suspension (phosphoric acid buffer solution, pH 7) containing 4 g. of Saccharomyces cerevisiae was added and stirring was made for 7 minutes. The polyvinyl alcohol concentration of the resulting aqueous suspension was 2.7 wt.% and the suspended bentonite concentration thereof was 3.1 wt.%. On the other hand, from the results of analysis of the aforesaid bentonite (X-ray diffractiometry, differential thermal analysis, identification through an electron microscope, heat dehydration, interlaminar expansion by glycerin, cation exchange: 137 meq/100g, chemical analysis: $SiO_2$ 71, $Al_2O_3$ 9, $Fe_2O_3$ 3, CaO 0.5, MgO 3, $TiO_2$ 0.5, $Na_2O$ 4, $K_2O$ 0.5, $M_nO$ 0.0, $P_2O_5$ 0.0, $H_2O$ 8.5 wt.%), its dry clay-minerals composition proves to be montmorillonite group 66, illites 1, talc 1, pyrophyllite 14 and vermiculite 1.

Therefore, the concentration of three-layer type clay minerals in the above aqueous suspension is 2.6 wt.%, and the amount of the clay minerals used is equal to that of the polyvinyl alcohol. 180 g. of this aqueous suspension was poured in a sterile room into a mold for 600 pcs. hollow cylinders (Raschig rings) each 8mm in outside diameter, 4mm in inside diameter and 8mm long and cooled (freeze-dehydrated) at -50°C for 0.5 hour, then the upper cover of the mold was removed and a 6 hours' vacuum-dehydration was applied to the lower cover supporting the moldings (Raschig rings). After thawing, there were obtained 53 g. of very elastic gel moldings (water content 75 wt.%, dehydration percentage 71 wt.%). The Raschig rings thus obtained were immersed in 84 ml. of a pre-sterilized 0.9% saline solution for 10 hours; as a result, the weight thereof increased to 64 g. (water content 78 wt.%). The aforesaid yeast was not detected from this immersion solution. Then, 40 ml. of an alcohol preparing aqueous substrate solution comprising 5 wt.% of glucose and 60 ppm of magnesium sulfate heptahydrate was charged into a Sakaguchi flask (500 ml.), then sterilized at 120°C for 25 minutes and allowed to cool in a sterile room. Thereafter, 4.5 g. (corresponding to 45 pieces) of the above gel moldings (Raschig rings) were added therein and the flask, after putting a burnt cotton plug, was shaken in a thermostatic room at 28-31°C. As a result, after 18 hours, 2.0 wt.% of ethyl alcohol was detected from this substrate (aqueous solution). There was recognized no exudation of the yeast into the substrate.

On the other hand, 40 ml. of the above aqueous substrate solution was added into 4.5 g. of a suspension containing 0.4 g. of Saccharomyces cerevisiae of the same strain as the aforesaid yeast, and the resulting aqueous suspension was shaken in the same way as above. As a result, after 18 hours, the ethyl alcohol concentration of the aqueous suspension reached 2.1 wt.% (82% of theory). It is therefore apparent that the yeast embedded in the Raschig rings of the present invention was kept immobilized therein throughout the operation of alcoholic fermentation and that its glycolysis activity (ethyl alcohol producing ability) reaches 95% of the original yeast (unimmobilized yeast).

The foregoing commercial bentonite powder (2.0 g.) was subjected to a swelling force test as defined by the Pharmacopoeia of Japan; that is, 100 ml. of water was charged into a graduated measuring cylinder and the powder was added in ten stages in such a manner that after almost all quantity of a previously added sample had precipitated, the next sample was added. After addition of the whole quantity of the powder, the cylinder was allowed to stand for 24 hours; as a result, the apparent volume of the precipitate was only 9 ml. much lower than the defined value of 20 ml. (or higher). Likewise, the bentonite powder (6.0 g.) was subjected to a gel forming ability test as defined by the Pharmacopoeia of Japan; that is, the powder was mixed with 0.30 g. of magnesium oxide and then added into 200 ml. of water. After shaking for 1 hour, 100 ml. of the resulting suspension was sampled and allowed to stand for 24 hours, but the quantity of a transparent liquid separated as an upper layer reached 4 ml. larger than the defined value of 2 ml. (or less).

Thus, the commercial bentonite used in this Example does not satisfy what is defined by the Pharmacopoeia of Japan, but it causes no trouble in the present invention as previously noted.

Example 7

87 g. powder (water content 7 wt.%) of a commercially available polyvinyl alcohol [degree of hydrolysis: 96 mol%, viscosity-average polymerization degree: 1,500, viscosity as a 4% aqueous solution: 24 cP (20°C)] was dissolved in 919 g. of water to prepare an 8 wt.% aqueous solution (pH 6.9).

120 g. of a commercial vermiculite powder (water content 9 wt.%) was dispersed in 1,200 g. of water to obtain an 8 wt.% aqueous suspension (pH 6.6). 190 g. of the above polyvinyl alcohol solution and also 190 g. of the vermiculite suspension just prepared were mixed, then sterilized with steam under pressure at 120°C for 20 minutes and subsequently allowed to cool in a sterile room. Thereafter, 20 ml. of a suspension (phosphoric acid buffer solution, pH 7) containing 4 g. of Candida sp. was added therein and stirring was made for 7 minutes to give an aqueous suspension containing 3.8 wt.% of polyvinyl alcohol and also 3.8 wt.% of vermiculite. On the other hand, from the results of analysis of vermiculite (powder), its dry clay-minerals composition (wt.%) proves to be montmorillonite group 4, illites 2, talc 3, pyrophyllite 2 and vermiculite 85 ($SiO_2$ 42, $Al_2O_3$ 19, $TiO_2$ 2, $Fe_2O_3$ 8, FeO 2, CaO 1, MgO 22, $K_2O$ 1, $Na_2O$ 1). Therefore, the concentration of three-layer type clay minerals in the above aqueous suspension is 3.5 wt.% and the amount of the clay minerals used is about the same as that of the polyvinyl alcohol. 400 g. of this aqueous suspension was poured into a Raschig ring (8mm x 8mm) forming mold (for 1,300 pieces) in a sterile room and cooled (freeze-molded) at -65°C for 0.5 hour, then the upper cover of the mold was removed and a 6 hours' vacuum-dehydration was applied to the lower cover supporting the moldings (Raschig rings). After thawing, 76 g. of elastic gel moldings (water content 55 wt.%, dehydration percentage 81 wt.%) were obtained. When the moldings were immersed in 100 ml. of a pre-sterilized 0.9% saline solution for 6 hours, their weight increased to 86 g. (water content 60 wt.%). The aforementioned yeast was not detected from this immersion solution.

50 ml. of an itaconic acid preparing aqueous substrate solution comprising 10% glucose, 0.1% ammonium chloride, 0.02% potassium primary phosphate, 0.05% magnesium sulfate heptahydrate and 0.05% yeast extract was charged into a Sakaguchi flask (500 ml.), then sterilized at 120°C for 20 minutes and subsequently allowed to cool in a sterile room. Thereafter 120 pieces (8 g.) of the above Raschig rings were introduced therein and the flask, after putting a burnt cotton plug, was shaken in a thermostatic room at 24-27°C. Because of a gradual decrease of the pH value of the aqueous substrate solution, the operation for neutralization (pH adjustment to 6.0) with 1N potassium hydroxide was performed twice a day. After 5 days, 2.0 wt.% of itaconic acid was recognized in the aqueous substrate solution, its yield was 28 mol%. Exudation of the yeast into the aqueous substrate solution was not recognized.

On the other hand, 5.0 ml. of the aforesaid aqueous substrate solution was added into 8 g. of a suspension (phosphoric acid buffer solution pH 7) containing 0.4 g. of Candida yeast of the same strain as the above yeast, followed by shaking and pH adjustment in the same manner. After 5 days, the itaconic acid concentration of the aqueous substrate solution was 2.0 wt.% (28 mol% of theory).

Therefore, it is apparent that the yeast embedded in the Raschig rings of the present invention was wholly immobilized in the gel and that its itaconic acid forming activity can be regarded as being equal to that of the original yeast.

Example 8

An 8.0 wt.% aqueous polyvinyl alcohol solution was prepared in the same way as in Example 6.

30 g. of the bentonite powder used in Example 6 was dispersed in 560 g. of an aqueous sodium pyrophosphate solution ($Na_4P_2O_7$ 10 $H_2O$ 10.7 wt.%) to obtain a 4.5 wt.% aqueous bentonite suspension (pH 10.6), to which was added 9 ml. of 6N sulfuric acid to adjust the pH value of the aqueous suspension to 6.9.

110 g. of the above aqueous polyvinyl alcohol solution and 60 g. of the above bentonite suspension were mixed, then sterilized with steam under pressure at 120°C for 6 hours and subsequently allowed to cool in a sterile room. Thereafter, 20 g. of a suspension (phosphoric acid buffer solution) containing 4 g. of Leuconostoc mesenteroides was added therein and stirring was made for 7 minutes to prepare an aqueous suspension containing 4.5 wt.% polyvinyl alcohol and 1.5 wt.% bentonite. In this case, moreover, the concentration of suspended three-layer type clay minerals was 1.2 wt.% and the amount of the clay minerals used was one-fourth of that of the polyvinyl alcohol.

This aqueous suspension (190 g.) was poured into a Raschig ring (8mm x 8mm) forming mold (for 630 pieces) in a sterile room and cooled (freeze-molded) at -47°C for 0.5 hour, then the upper cover of the mold was removed and a 9 hours' vacuum-dehydration was applied to the lower cover supporting the moldings (Raschig rings). After thawing, 49 g. of very elastic gel moldings (water content 68 wt.%, dehydration percentage 74 wt.%) were obtained. When this gel was immersed in 60 ml. of a pre-sterilized 0.9% saline solution for 6 hours, it absorbed water and increased in weight to 58 g. (water content 73 wt.%).

58 g. of the above Raschig rings were charged irregularly into a cylindrical column made of acrylic resin 3 cm in diameter by 60 cm height and an L-citrulline preparing aqueous substrate solution (L-arginine hydrochloride 0.6%, acetic acid 0.25%, sodium acetate 13%, calcium chloride 1%, pH 6.5, 30°C) which had been sterilized at 120°C for 20 minutes was introduced from the bottom of the column at a rate of 23 ml/h. As a result, after 37 hours, the concentration of L-citrulline (L-$\alpha$-amino-$\delta$-carbamidovaleric acid) in the effluent reached 0.02 mol/$\ell$ (0.35 wt.%).

Example 9

86 g. powder (water content 7 wt.%) of a commercially available polyvinyl alcohol [degree of hydrolysis: 97 mol%, viscosity-average polymerization degree: 1,500, viscosity as a 4% aqueous solution: 24 cP (20°C)] was dissolved in 914 g. of water to prepare an 8.0 wt.% aqueous solution (pH 6.8).

104 g. of a commercially available bentonite (powder for reagent, water content 15 wt.%) was dispersed in 1,500 g. of water to prepare a 5.5 wt.% bentonite suspension (pH 10.7), into which was added 4 ml. of sulfuric acid to adjust the pH value of the suspension to 6.8.

120 g. of the above aqueous polyvinyl alcohol solution and 50 g. of the above bentonite suspension were mixed, then sterilized with steam under pressure at 120°C for 6 hours and subsequently allowed to cool in a sterile room. Thereafter, 20 g. of a suspension (phosphoric acid buffer solution) containing 4 g. of Eruvinia herbicola was poured into the mixed aqueous suspension and stirring was made for 7 minutes. The concentration of polyvinyl alcohol and the suspended bentonite concentration in the resulting aqueous suspension were 5 wt.% and 1.5 wt.%, respectively. On the other hand, from the results of analysis of the above bentonite (powder) (X-ray diffractiometry, differential thermal analysis, identification through an electron microscope, heat dehydration, interlaminar expansion by glycerin, cation exchange: 89 meq/100g, chemical analysis: $SiO_2$ 66.7, $Al_2O_3$ 8.7, $Fe_2O_3$ 3.1, CaO 0.3, MgO 1.6, $Na_2O$ 3.2, $K_2O$ 0.3, $TiO_2$ 0.0, MnO 0.0, $P_2O_5$ 0.0, $H_2O$ 15 wt.%), its dry clay-minerals composition proves to be montmorillonite group 64, illites 1, talc 3, pyrophyllite 18, vermiculite 1. Therefore, the concentration of three-layer type clay minerals in the above aqueous suspension is 1.3 wt.% and the amount of the clay minerals used is one-fourth of the amount of polyvinyl alcohol.

190 g. of this aqueous suspension was poured into a Raschig ring (8mm x 8mm) forming mold (for 630 pieces) in a sterile room and cooled (freeze-molded) at -70°C for 0.5 hour, then the upper cover of the mold was removed and an 8 hours' vacuum-dehydration was applied to the lower cover supporting the moldings (Raschig rings). After thawing, 39 g. of very elastic gel moldings (water content 58 wt.%, dehydration percentage 79 wt.%) were obtained. When this molded gel was immersed in 80 ml. of a pre-sterilized 0.9% saline solution for 6 hours, it absorbed water and increased in weight to 48 g. (water content 66%). The aforesaid bacterium was not detected from this immersion solution.

48 g. of the above gel moldings were charged irregularly into a porcelain column 3 cm in diameter by 60 cm height and a tyrosine preparing aqueous substrate solution (phenol 0.1%, sodium nitrite 0.2%, ammonium acetate 5%, sodium pyruvate 3%, pyridoxal phosphate 100 ppm, ethylenediamine tetraacetate 300 ppm, pH 8, 30°C) was introduced from the bottom of the column at a rate of 20 ml/h. As a result, after 12 hours, the concentration of $\beta$-tyrosine [$\beta$-(p-hydroxyphenyl)alanine] in the effluent reached 620 ppm.

The commercial bentonite powder (2.0 g.) used in this Example was subjected to a swelling test as defined by the Pharmacopoeia of Japan, but the apparent volume of the precipitate was only 8 ml. much less than the defined value of 20 ml. (or more). Likewise, a gel forming ability test was conducted according to the Pharmacopoeia of Japan, but the amount of a transparent liquid separated as an upper layer reached 12 ml. much larger than the defined value of 2 ml.

(or less).

        Thus, the commercial bentonite used in this Example does not satisfy what is defined by the Pharmacopoeia of Japan, but, as previously noted, it can serve as a useful material for immobilizing bacteria.


Example 10

        133 g. powder (water content 8 wt.%) of a commercially available polyvinyl alcohol [degree of hydrolysis:  99.7 mol%, viscosity-average polymerization degree:  2,600, viscosity as a 4% aqueous solution:  64 cP (20°C)] was dissolved in 1,000 g.

of water to prepare an 11 wt.% aqueous solution (pH 6.8).

        78 g. of pyrophyllite (water content 6 wt.%, obtained in Mitsuishi, Okayama Prefecture) was dispersed in 1,000 g.

of water to prepare a 6.8 wt.% aqueous suspension (pH 6.5).

        90 g. of the above aqueous polyvinyl alcohol solution and 10 g. of the above aqueous pyrophyllite suspension were mixed, then sterilized with steam under pressure at 120°C for 5 hours and subsequently allowed to cool in a sterile room.

Thereafter, 20 g. of a suspension (phosphoric acid buffer solution) containing 4 g. of Arthrobacter oxydans was poured into the mixed aqueous suspension and stirring was made for 7 minutes.  The concentration of polyvinyl alcohol and the suspended pyrophyllite concentration in the resulting aqueous suspension were 8 wt.% and 0.6 wt.%, respectively. On the other hand, from the results of analysis of the pyrophyllite, its dry clay-minerals composition (wt.%) proves to be montmorillonite group 4, illite 0, talc 0, pyrophyllite 83 and vermiculite 3 ($SiO_2$ 66, $Al_2O_3$ 29, $TiO_2$ 0.04, $Fe_2O_3$ 0.2, FeO 0.1, MgO 0.5, CaO 0.4, $Na_2O$ 0.03, $P_2O_5$ + $K_2O$ trace). Therefore, the concentration of three-layer type clay minerals in the above aqueous suspension is 0.5 wt.% and the amount of the clay minerals used is 1/16 of the amount of polyvinyl alcohol. 120 g. of this aqueous suspension was poured into a Raschig ring (8mm x 8mm) forming mold (for 400 pieces) and cooled (freeze-molded) at -65°C for 0.5 hour, then the upper cover of the mold was removed and the lower cover thereof supporting the moldings was subjected to vacuum-dehydration for 5 hours. After thawing, 59 g. of a molded gel (water content 75 wt.%, dehydration percentage 51 wt.%) was obtained. When this molded gel was immersed in 40 ml. of a pre-sterilized 0.9% saline solution for 6 hours, it absorbed water and increased in weight to 70 g. (water content 79 wt.%). The aforesaid bacterium was not detected from this immersion solution.

50 ml. of a lactic acid preparing aqueous substrate solution comprising 5% 1,2-propanediol and 0.06M phosphoric acid buffer solution (pH 8.0) was charged into a Sakaguchi flask (500 ml.), then sterilized at 120°C for 20 minutes and allowed to cool in a sterile room. Thereafter, the above molded gel was introduced therein and the flask, after putting a burnt cottom plug, was shaken in a thermostatic room at 28-32°C.

After 24 hours, the lactic acid concentration of the aqueous substrate solution was 0.4% (yield: 6.7 mol%). Exudation of the bacterium into the aqueous substrate solution was not recognized.

On the other hand, 50 ml. of the above aqueous substrate solution was added into 4 g. of a suspension (phosphoric acid buffer solution, pH 7) containing 0.4 g. of Arthrobacter oxydans of the same strain as the above bacterium, and subsequent operations were the same as above. As a result, after 24 hours, the lactic acid concentration of the aqueous substrate solution was 0.4% (yield: 6.7 mol%). Thus, the bacterium embedded in the gel of the present invention exhibits an activity equal to that of the original unbedded bacterium.

Example 11

1,040 g. powder (water content 8 wt.%) of a commercially available polyvinyl alcohol [degree of hydrolysis: 97 mol%, viscosity-average polymerization degree: 1,700, viscosity as a 4% aqueous solution: 27 cP (20°C)] was dissolved in 8,000 g. of water to prepare a 10.6 wt.% aqueous solution (pH 6.8). Then, 68 g. of a commercially available active white earth (water content 11 wt.%) was added into this aqueous solution followed by stirring for 25 minutes to obtain an aqueous suspension (pH 3.5), into which was added 26 ml. of 2N sodium hydroxide to adjust the pH of the suspension to 6.8. Thereafter, the aqueous suspension was sterilized with steam under pressure at 120°C for 30 minutes and allowed to cool in a sterile room, then 20 g. of a suspension (phosphoric acid buffer solution) containing 4 g. of Acetobacter suboxydans was poured into the above aqueous suspension followed by stirring for 7 minutes. The polyvinyl alcohol concentration and the concentration of the active white earth in the resulting aqueous suspension are 10 wt.% and 0.6 wt.%, respectively. On the other hand, from the results of analysis of the active white earth, its dry clay-minerals composition (wt.%) proves to be montmorillonite group 48, illites 7, talc 4, pyrophyllite 14, vermiculite 2 and kaolinite + halloysite 17 ($SiO_2$ 74, $Al_2O_3$ 18, $Fe_2O_3$ 1, MgO 2, CaO 4, $Na_2O$ + $K_2O$ 1). Therefore, the concentration of three-layer type clay minerals in the above aqueous suspension is 0.45 wt.% and it is 1/23 of the concentration of polyvinyl alcohol.

220 g. of the aqueous suspension was poured into a Raschig ring (8mm x 8mm) forming mold (for 730 pieces) in a sterile room and cooled (freeze-molded) at -45°C for 0.5 hour, then the upper cover of the mold was removed and the lower cover thereof supporting the moldings was subjected to vacuum-dehydration for 84 hours. After thawing, 66 g. of a molded gel (water content 59 wt.%, dehydration percentage 70 wt.%) was obtained. When this molded gel was immersed in 80 ml. of a pre-sterilized 0.9% saline solution for 6 hours, it absorbed water and increased in weight to 74 g. (water content 63 wt.%).

74 g. of the above Raschig rings were charged irregularly into the column described in Example 9 and a sorbose preparing aqueous substrate solution (D-sorbitol 5%, corn steep liquor 0.1%, pH 6.5, 30°C) which had been sterilized at 120°C for 20 minutes was introduced from the bottom of the column at a rate of 23 ml/h. As a result, after 17 hours, the concentration of L-sorbose in the effluent reached 3.6% (yield: 71 mol%).

Example 12

81 g. powder (water content 9 wt.%) of a commercially available polyvinyl alcohol [degree of hydrolysis: 99.7 mol%, viscosity-average polymerization degree: 2,600, viscosity as a 4% aqueous solution: 64 cP (20°C)] was dissolved in 1,130 g. of water to prepare a 6.1 wt.% aqueous solution (pH 6.9). Separately, 127 g. of a commercially available acid clay (water content 12 wt.%) was dispersed in 1,230 g. of water to obtain an 8.2 wt.% aqueous acid clay suspension (pH 6).

140 g. of the above aqueous polyvinyl alcohol solution and 20 g. of the above aqueous acid clay suspension were mixed, then sterilized with steam under pressure at 120°C for 30 minutes and allowed to cool in a sterile room. Thereafter, 20 g. of a suspension (phosphoric acid buffer solution) containing 4 g. of Serratia marcescens was poured into the mixed aqueous suspension followed by stirring for 7 minutes. The polyvinyl alcohol concentration and the concentration of the acid clay in the resulting aqueous suspension are 4.7 wt.% and 0.9 wt.%, respectively. On the other hand, from the results of analysis of the acid clay, its dry clay-minerals composition (wt.%) proves to be montmorillonite group 46, illites 6, talc 1, pyrophyllite 14, vermiculite 4 and kaolinite + halloysite 23 ($SiO_2$ 69, $Al_2O_3$ 18, CaO 3, MgO 1.5, Na O 0.1, $K_2O$ 0.1, $Fe_2O_3$ 2). Therefore, the concentration of three-layer type clay minerals in the above aqueous suspension is 0.6 wt.% and it is 1/8 of the concentration of polyvinyl alcohol.

180 g. of the aqueous suspension was poured into a Raschig ring (8mm x 8mm) forming mold (for 600 pieces) in a sterile room and cooled (freeze-molded) at -63°C for 0.5 hour, then the mold was disjointed and the molded articles were taken out and vacuum-dehydrated for 6 hours. After thawing, 77 g. of a molded gel (water content 82 wt.%, dehydration percentage 57 wt.%) was obtained. When this molded gel was immersed in 80 ml. of a pre-sterilized 0.9% saline solution for 6 hours, it absorbed water and increased in weight to 84 g. (water content 83 wt.%). The aforesaid bacterium was not detected from this immersion solution.

84 g. of the above Raschig rings were charged irregularly into the column described in Example 9 and an L-isoleucine preparing aqueous substrate solution (glucose 3%, D-threonine 1.5%, magnesium sulfate heptahydrate 0.05%, urea 0.8%, phosphoric acid buffer solution 0.06M, pH 7, 30°C) which had been sterilized at 120°C for 20 minutes was introduced from the bottom of the column at a rate of 25 ml/h. As a result, after 35 hours, the concentration of L-isoleucine (L-$\alpha$-aminocaproic acid) in the effluent reached 0.6 wt.% (37% of theory).

Example 13

87 g. powder (water content 7 wt.%) of a commercially available polyvinyl alcohol [degree of hydrolysis: 96 mol%, viscosity-average polymerization degree: 1,700, viscosity as a 4% aqueous solution: 27 cP (20°C)] was dissolved in 920 g.

of water to prepare an 8 wt.% aqueous solution (pH 7.0).

Separately, 120 g. of a commercially available talc (water content 14 wt.%) was dispersed in 1,190 g. of water to obtain an 8 wt.% aqueous talc suspension (pH 8).

350 g. of the above aqueous polyvinyl alcohol solution and 30 g. of the above aqueous talc suspension were mixed, then sterilized with steam under pressure at 120°C for 20 minutes and allowed to cool in a sterile room. Thereafter, 20 ml. of a suspension (phosphoric acid buffer solution, pH 7) containing 4 g. of Acetobacter suboxydans was added and stirring was made for 7 minutes. The concentration of polyvinyl alcohol and that of talc in the resulting aqueous suspension are 7 wt.% and 0.6 wt.%, respectively.

On the other hand, from the results of analysis of the talc (powder), its dry clay-minerals composition (wt.%) proves to be montmorillonite group 6, illites 2, talc 87, pyrophyllite 2 and vermiculite 1 ($MgO$ 32, $SiO_2$ 64, $Al_2O_3$ 1, $CaO$ 0.3, $K_2O$ 0.3, $Na_2O$ 0.7, $Fe_2O_3$ 0.5). Therefore, the concentration of three-layer type clay minerals in the above aqueous suspension is 0.6 wt.% and it is 1/12 of the concentration of polyvinyl alcohol.

400 g. of this aqueous suspension was poured into a Raschig ring (8mm x 8mm) forming mold (for 1,330 pieces) in a sterile room and cooled (freeze-molded) at -75°C for 0.5 hour, then the mold was disjointed and the molded articles were taken out and vacuum-dehydrated for 8 hours. After thawing, 98 g. of a molded gel (water content 65 wt.%, dehydration percentage 75 wt.%) was obtained. When this molded gel was immersed in 100 ml. of a pre-sterilized 0.9% saline solution
for 6 hours, it absorbed water and increased in weight to 105 g.
(water content 67 wt.%).  The aforesaid bacterium was not
detected from this immersion solution.

105 g. of the above gel moldings were charged
irregularly into the column described in Example 9 and a
dihydroxyacetone preparing aqueous substrate solution (glycerin
2.5%, corn steep liquor 0.1%, pH 6.5, 30°C) which had been
sterilized at 120°C for 20 minutes was introduced from the bottom
of the column at a rate of 47 ml/h.  As a result, after 24
hours, the concentration of dihydroxyacetone in the effluent
was 1.2 wt.% (yield:  50 mol%).

Example 14

85 g. powder (water content 6 wt.%) of a commercially
available polyvinyl alcohol [degree of hydrolysis:  97 mol%,
viscosity-average polymerization degree:  2,200, viscosity as
a 4% aqueous solution:  54 cP (20°C)] was dissolved in 915 g.
of water to prepare an 8.0 wt.% aqueous solution (pH 6.9).

106 g. of a commercially available bentonite (powder
for reagent, water content 17 wt.%) was dispersed in 1,490 g.
of water to obtain a 5.5 wt.% aqueous bentonite suspension
(pH 10.1), into which was added 4 ml. of 6N sulfuric acid to
adjust the pH value of the suspension to 6.6.

380 g. of the above aqueous polyvinyl alcohol
solution and 20 g. of the above bentonite suspension were
mixed, then sterilized with steam under pressure at 120°C
for 6 hours and allowed to cool in a sterile room.  Thereafter, 20 ml. of a suspension [tris(hydroxymethyl)aminomethane buffer solution, pH 8.0] containing 4 g. of Lactobacillus brevis was added and stirring was made for 7 minutes. The polyvinyl alcohol concentration and the suspended bentonite concentration of the resulting aqueous suspension are 7 wt.% and 0.25 wt.%, respectively. On the other hand, from the results of analysis of the bentonite (powder) (X-ray diffractiometry, differential thermal analysis, identification through an electron microscope, heat dehydration, interlaminar expansion by glycerin, cation exchange: 78 meq/100g, chemical analysis: $SiO_2$ 67.2, $Al_2O_3$ 6.8, $Fe_2O_3$ 41, CaO 0.3, MgO 1.6, $TiO_2$ 0.4, MnO 0.1, $P_2O_5$ 0.1, $Na_2O$ 3.2, $K_2O$ 0.4 wt.%), its dry clay-minerals composition proves to be montmorillonite group 58, illites 1, talc 1, pyrophyllite 11 and vermiculite 1. Therefore, the concentration of three-layer type clay minerals in the above aqueous suspension is 0.17 wt.% and it is 1/40 of the concentration of polyvinyl alcohol.

420 g. of this aqueous suspension was poured into a Raschig ring (8mm x 8mm) forming mold (for 1,400 pieces) in a sterile room and cooled (freeze-molded) at -68°C for 5 hours, then the mold was disjointed and the molded articles were taken out and vacuum-dehydrated for 7 hours. After thawing, 160 g. of a molded gel (water content 78 wt.%, dehydration percentage 62 wt.%) was obtained.

The molded gel was immersed in 100 ml. of a pre-sterilized 0.9% saline solution for 6 hours; as a result, it absorbed water and increased in weight to 182 g. (water content 80 wt.%). The aforesaid bacterium was not detected from this immersion solution.

182 g. of the above gel moldings were charged irregularly into the column described in Example 9 and a fructose preparing aqueous substrate solution (glucose 5.0 wt.%, manganese sulfate tetrahydrate 0.01 mol/$\ell$, 62°C, pH 6.3) which had been sterilized at 120°C for 20 minutes was introduced from the bottom of the column at a rate of 58 ml/h. As a result, after 70 hours, the effluent proved to have a pH value of 6.7 and a fructose concentration of 2.0 wt.% (yield: 40 mol%).

The commercial bentonite powder (2.0 g.) used in this Example was subjected to the foregoing swelling test according to the Pharmacopoeia of Japan, but the apparent volume of the precipitate was only 9 ml. much less than the defined value of 20 ml. (or more). Likewise, the foregoing gel forming ability test was made for this bentonite powder according to the Pharmacopoeia of Japan, and in this case the amount of a transparent liquid separated as an upper layer barely reached the defined value of 2 ml. (or less).

Thus, none of the three commercial bentonites used in Examples 6, 9 and 14 satisfy what is defined by the Pharmacopoeia of Japan, but in the present invention, as previously noted, they can serve as useful materials for immobilizing microorganism.

Example 15

In Example 14, Streptomyces phaeochromogenes was used in place of Lactobacillus brevis in an amount of 1/200 of the amount used in Example 14, that is, 20 ml. of a suspension (culture medium) containing 0.02 g. of the bacterium was used. 420 g. of a mixed suspension of the polyvinyl alcohol, the bentonite and the bacterium was freeze-molded and dehydrated in the same manner to obtain 158 g. of a molded gel (water content 78 wt.%, dehydration percentage 44 wt.%). When the molded gel was immersed in 100 ml. of a pre-sterilized 0.9% saline solution for 6 hours, it absorbed water and increased in weight to 183 g. (water content 80 wt.%). It was washed with 100 ml. of a pre-sterilized 0.9% saline solution. The gel moldings (183 g.) were charged irregularly into the column described in Example 9 and a culture solution (xylose 1%, peptone 1%, meat extract 1%, yeast extract 0.3%, common salt 0.5%, magnesium sulfate hydrate 0.06%, 30°C) which had been sterilized at 120°C for 20 minutes was introduced from the bottom of the column over a 24 hour period at a rate of 53 ml/h. Before and after this operation for introducing the culture solution (medium), part of the packing (gel) was sampled and observed with a scanning electron microscope. As a result, before the said operation, the bacterium was little recognized in the gel, but after the operation, a bacterial colony could be recognized easily.

Then, a fructose preparing substrate solution (glucose 5%, magnesium sulfate 0.01M, pH 8.3, 65°C) was introduced from the bottom of the column at a rate of 30 ml/h. As a result, after 100 hours, the fructose concentration of the effluent reached 2 wt.% (yield: 40 mol%) and the pH value thereof was 6.4.

On the other hand, after the formation of the gel, the above-mentioned culture operation was omitted and the substrate solution was introduced immediately over a 200 hour period, but in this case the fructose concentration of the effluent proved to be only 0.02 wt.%.

Thus, in the present invention, a live microorganism can be immobilized without damage thereto and therefore, as a matter of course, it is apparent that the live microorganism propagates remarkably in a culture medium.

Example 16

85 g. powder (water content 6 wt.%) of a commercially available polyvinyl alcohol [degree of hydrolysis: 97 mol%, viscosity-average polymerization degree: 2,200, viscosity as a 4% aqueous solution: 54 cP (20°C)] was dissolved in 914 g. of water to prepare an 8.0 wt.% aqueous solution (pH 6.8).

Separately, 98 g. of illite (water content 8 wt.%, obtained in Ota City, Shimane Prefecture) was dispersed in 1,200 g. of water to obtain an 8 wt.% aqueous illite suspension (pH 7.4).

370 g. of the above aqueous polyvinyl alcohol solution and 20 g. of the above aqueous illite suspension were mixed, then sterilized with steam under pressure at 120°C for 2 hours and allowed to cool in a sterile room. Thereafter, 20 ml. of a suspension (phosphoric acid buffer solution, pH 7) containing 4 g. of Gluconobacter suboxydans was added and stirring was made for 7 minutes. The concentration polyvinyl alcohol and that of illite in the resulting aqueous suspension are each 7.3 wt.%. On the other hand, from the results of analysis of the illite (powder), its dry clay-minerals composition (wt.%) proves to be montmorillonite group 1, illites 87, talc 1, pyrophyllite 6 and vermiculite 1 ($SiO_2$ 52, $Al_2O_3$ 24, $Fe_2O_3$ 3, FeO 3, MgO 4, CaO 1, $K_2O$ 7, $Na_2O$ 1, $TiO_2$ 1, $Mn_2O_3$ 2). Therefore, the concentration of three-layer type clay minerals in the above aqueous suspension is 0.36 wt.% and it is 1/20 of the concentration of polyvinyl alcohol.

410 g. of the aqueous suspension was poured into a Raschig ring (8mm x 8mm) forming mold (for 1,360 pieces) in a sterile room and cooled (freeze-molded) at 54°C for 0.5 hour, then the upper cover of the mold was removed and the lower cover thereof supporting the moldings was subjected to vacuum-dehydration for 6 hours. After thawing, 230 g. of a molded gel (water content 85 wt.%, dehydration percentage 44 wt.%) was obtained. The molded gel was immersed in 100 ml. of a pre-sterilized 0.9% saline solution for 6 hours; as a result, it absorbed water and its weight increased to 268 g. (water content 87 wt.%). The aforesaid bacterium was not detected from this immersion solution.

268 g. of above gel moldings were charged irregularly into the column described in Example 9 and a D-xylose preparing aqueous substrate solution (D-arabitol 5%, magnesium heptahydrate 0.02%, potassium sulfate buffer solution 0.06M, pH 5, 35°C) which had been sterilized at 120°C for 20 minutes was introduced from the bottom of the column at a rate of 58 ml/h. As a result, after 24 hours, the concentration of D-xylose in the effluent was 3.5 wt.% (yield: 40 mol%).

Thus, as shown in Examples 6 through 16, it is apparent that all of the three-layer type (2:1 type) clay minerals used therein are useful as a carrier for immobilizing microorganisms in the present invention.

Example 17

104 g. powder (water content 8 wt.%) of a commercially available polyvinyl alcohol [degree of hydrolysis: 9.7 mol%, viscosity-average polymerization degree: 1,700, viscosity as a 4% aqueous solution: 27 cP (20°C)] was dissolved in 800 g. of water to prepare a 10.6 wt.% aqueous solution thereof (pH 6.8).

60 g. of silicic acid anhydride, 40 g. of magnesium oxide, 42 g. of sodium fluoride and 26 g. of lithium fluoride were mixed, then transferred into a platinum crucible and stoppered tightly. The mixture was allowed to melt in an electric furnace at 1,385°C for 6 hours and then cooled slowly down to 810°C at a cooling rate of 25°C/min. The resulting solid matter was further allowed to cool to room temperature and then pulverized to powder finer than 100 mesh (147 $\mu$m). By classification, 81 g. of fine powder (110°C dry powder) of 200 mesh (74 $\mu$m) or finer was obtained.

The result of chemical analysis of this powder is as follows (wt.%): Na 5.6, Mg 12.8, Li 1.7, Si 28.9, O 41.2, F 9.8, and there was obtained an experimental formula $Na_{0.95}Mg_{2.05}Li_{0.95}(Si_4O_{10})F_2$ nearly conforming to sodium taeniolite $NaMg_2Li(Si_4O_{10})F_2$. According to the result of X-ray diffractiometry of the powder, there was not recognized a crystalline substance other than sodium taeniolite (fluorotetrasilicon mica), but due to changing of the crystal structure into amorphous state in the course of the above
pulverization, the content of illites (three-layer type clay
minerals) proved to be 63%.

80 g. of the above sodium taeniolite was dispersed
in 1,170 g. of water to obtain a 6.4 wt.% synthetic illite
(mica) suspension (pH 7).

190 g. of the above aqueous polyvinyl alcohol
solution and also 190 g. of the above synthetic illite suspension
were mixed, then sterilized with steam under pressure at 120°C
for 20 minutes and allowed to cool in a sterile room. Thereafter,
20 ml. of a suspension (phosphoric acid buffer solution, pH 7)
containing 4 g. of Acetobacter aceti was added and stirring
was made for 7 minutes. The polyvinyl alcohol concentration
and that of the suspended synthetic illite in the resulting
aqueous suspension are 5 wt.% and 3 wt.%, respectively. The
concentration of three-layer type clay minerals in the same
solution is 2 wt.% and it is 2/5 of the concentration of polyvinyl
alcohol.

400 g. of this aqueous suspension was poured into
a Raschig ring (8mm x 8mm) forming mold (for 1,330 pieces)
in a sterile room and cooled (freeze-molded) at -73°C for
0.5 hour, then the upper cover of the mold was removed and
the lower cover thereof supporting the moldings was subjected
to vacuum-dehydration for 6 hours. After thawing, 233 g. of
a molded gel (water content 92 wt.%, dehydration percentage
42 wt.%) was obtained. The molded gel was immersed in 100 ml.
of a pre-sterilized 0.9% saline solution for 4 hours; as a
result, it absorbed water and its weight increased to 270 g.

(water content 93 wt.%). The aforementioned bacterium was not detected from this immersion solution.

50 ml. of a gluconic acid preparing aqueous substrate solution comprising 10% glucose, 0.2% corn steep liquor and 0.06M phosphoric acid buffer solution (pH 7) was charged into a Sakaguchi flask (500 ml.), then sterilized at 120°C for 20 minutes and allowed to cool in a sterile room. Thereafter, 11 g. of the above gel was put therein and the flask, after putting a burnt cotton plug, was shaken in a thermostatic room at 30-33°C. After 24 hours, the gluconic acid concentration of the aqueous substrate solution was 9.4 wt.% (yield: 87 mol%). Exudation of the bacterium into the aqueous substrate solution was not recognized.

On the other hand, 50 ml. of the above aqueous substrate solution was added into 11 g. of an aqueous suspension (phosphoric acid buffer solution, pH 7) containing 0.4 g. of Acetobacter aceti of the same strain as the aforementioned bacterium, and subsequent operations were the same. As a result, after 24 hours, the gluconic acid concentration of the aqueous substrate solution was 9.7 wt.% (yield: 89 mol%). Therefore, the bacterium embedded in the gel of the present invention exhibits an activity corresponding to 98% of the original unbedded bacterium.

That is, like the natural three-layer time clay-minerals used in Examples 6 through 16, the synthetic three-layer type clay minerals used in this Example are also useful in the present invention.

Example 18

86 g. powder (water content 7 wt.%) of a commercially available polyvinyl alcohol [degree of hydrolysis: 98.5 mol%, viscosity-average polymerization degree: 1,900, viscosity as a 4% aqueous solution: 31 cP (20°C)] was dissolved in 915 g. of water to prepare an 8.0 wt.% aqueous solution thereof (pH 7).

105 g. of a commercially available bentonite (powder for reagent, water content 16 wt.%) was dispersed in 1,490 g. of water to obtain a 5.5 wt.% aqueous suspension (pH 10.5), into which was added 3 ml. of 8N sulfuric acid to adjust the pH value of the suspension to 7.

60 g. of the above aqueous polyvinyl alcohol solution and 100 g. of the above aqueous bentonite suspension were mixed, then sterilized with steam under pressure at 120°C for 3 hours and allowed to cool in a sterile room. Thereafter, 20 ml. of a culture solution containing 20 mg. (on a dry matter basis) of Saccharomyces cerevisiae was added and stirring was made for 7 minutes. The polyvinyl alcohol concentration and the concentration of the suspended bentonite in the resulting aqueous suspension are 2.7 wt.% and 3 wt.%, respectively. On the other hand, from the results of analysis of the bentonite (powder) (X-ray diffractiometry, differential thermal analysis, identification through an electron microscope, heat dehydration, interlaminar expansion by glycerin, cation exchange: 156 meq/100g, chemical analysis: $SiO_2$ 66, $Al_2O_3$ 8, $Fe_2O_3$ 3, $CaO$ 0.5, $MgO$ 3, $TiO_2$ 0.5, $Na_2O$ 3.5, $K_2O$ 0.5, $MnO$ 0.1, $P_2O_5$ 0.0, $H_2O$ 15 wt.%), its dry clay-minerals composition (wt.%) proves to be montmorillonite group 68, illite 0, talc 1, pyrophyllite 3 and vermiculite 2.

Therefore, the concentration of three-layer type clay minerals in the above aqueous suspension is 2.5 wt.% and the amount of the clay minerals used is equal to the amount of polyvinyl alcohol.

90 g. of this aqueous suspension was poured in a sterile room into a mold for forming 300 pieces of hollow cylinders (Raschig rings) each 8mm in outside diameter, 4mm in inside diameter and 8mm long and cooled (freeze-molded) at -45°C for 0.5 hours, then the upper cover of the mold was removed and the lower cover thereof supporting the moldings (Raschig rings) was subjected to vacuum-dehydration for 6 hours. After thawing, 27 g. of a very elastic gel (moldings) (water content 75 wt.%, dehydration percentage 70 wt.%) was obtained. When this molded gel was immersed in 40 ml. of a pre-sterilized 0.9% saline solution, its weight increased to 32 g. (water content 78 wt.%). The aforesaid bacterium was not detected from this immersion solution.

32 g. of the above Raschig rings were charged irregularly into a glass column 2 cm in diameter by 20 cm height and a culture medium (glucose 10%, yeast extract 0.05%, malt extract 0.05%, peptone 0.1%, potassium chloride 1%, pH 5.5, 32°C) which had been sterilized at 120°C for 20 minutes was introduced from the bottom of the column over a 29 hour period at a rate of 50 ml/h. Before and after the operation for introducing the culture medium, part of the packing was sampled and observed with a scanning electron microscope; as a result, before the said operation, the yeast was not recognized in the gel, but after the operation a large yeast colony (200 $\mu$m in

diameter) could be recognized.

Next, an ethyl alcohol preparing aqueous substrate solution (glucose 10 wt.%, magnesium sulfate heptahydrate 60 ppm, pH 5.7, 31°C) was introduced from the bottom of the column as a rate of 30 ml/h. As a result, after 20 hours, the ethyl alcohol concentration of the effluent reached 4 wt.% (78% of theory).

When after freeze-dehydration of the Raschig rings (moldings) the aforesaid growth (culture) operation was omitted and the substrate solution was immediately introduced over a 20 hour period, the ethyl alcohol concentration of the effluent proved to be only 0.1 wt.%.

Example 19

86 g. powder (water content 7 wt.%) of a commercially available polyvinyl alcohol [degree of hydrolysis: 97 mol%, viscosity-average polymerization degree: 1,700, viscosity as a 4% aqueous solution: 26 cP (20°C)] was dissolved in 914 g. of water to prepare an 8.0 wt.% aqueous solution thereof.

44 g. of the aqueous polyvinyl alcohol solution was sterilized with steam under pressure at 120°C for 20 minutes and then allowed to cool in a sterile room. Thereafter, 4 g. of a suspension (phosphoric acid buffer solution, pH 7) containing 0.8 g. of Saccharomyces cerevisiae was poured into the aqueous solution followed by stirring for 7 minutes to give an aqueous suspension containing 7.3 wt.% of polyvinyl alcohol. 40 g. of this aqueous suspension was poured in a sterile room onto a projections-disposed plate made of polyethylene (height of

74 0060052

projection: 1 mm, projection density: 74,000 pcs/m$^2$, shape of projection: a 1.8mm-dia. cylinder, percentage total area occupied by projections: 20%, size of the plate: 48cm x 17cm) and spread uniformly with a spatula to a thickness of 0.7 mm. After cooling (freeze-molding) at -53°C for 0.5 hour, the resulting molded article was taken out and vacuum-dehydrated for 4 hours. After thawing, 4.8 g. of a molded gel (yeast-containing net) (water content 25 wt.%, dehydration percentage 88 wt.%) was obtained.

When this molded gel was immersed in 40 ml. of a pre-sterilized 0.9% saline solution for 6 hours, it absorbed water and increased in weight to 28.8 g. (water content 88 wt.%). The aforementioned yeast was not detected from this immersion solution, from which it is seen that almost all quantity of the yeast was embedded (entrapped) in the net-like gel.

26 g. of the above yeast net was charged irregularly into a glass column 3cm in diameter by 10cm high, and an ethyl alcohol preparing aqueous substrate solution (glucose 10 wt.%, magnesium sulfate heptahydrate 60 ppm, pH 6, 32°C) which had been sterilized at 120°C for 20 minutes was introduced from the bottom of the column at a rate of 50 ml/h. As a result, after 11 hours, the ethyl alcohol concentration of the effluent reached 4.8 wt.% (94% of theory). After this operation was continued for 12 days, the ethyl alcohol concentration of the effluent proved to be 4.6 wt.%.

Example 20

84 g. powder (water content 5 wt.%) of a commercially available polyvinyl alcohol [degree of hydrolysis: 98.4 mol%, viscosity-average polymerization degree: 1,800, viscosity as a 4% aqueous solution: 29.5 cP (20°C)] was dissolved in 916 g. of water to prepare an 8.0 wt.% aqueous solution thereof (pH 6.9).

18 g. of the aqueous polyvinyl alcohol solution was sterilized with steam under pressure at 120°C for 20 minutes and then cooled in a sterile room. Thereafter, 2 g. of a suspension (phosphoric acid buffer solution, pH 7) containing 0.4 g. of Saccharomyces cerevisiae was poured into the aqueous solution followed by stirring for 7 minutes to give an aqueous suspension containing 7.2 wt.% of polyvinyl alcohol.

18 g. of the aqueous suspension was poured in a sterile room onto a projections-disposed plate made of polyethylene (height of projection: 2 mm, projection density: 300,000 pcs/m$^2$, shape of projection: a 5.6mm-dia. cylinder, percentage total area occupied by projections: 75%, size of the plate: 20cm x 20cm) and spread uniformly with a spatula to a thickness of 1.8 mm, followed by cooling (freeze-molding) at -53°C for 2 hours and subsequent vacuum-dehydrating for 5 hours. After thawing, 2.1 g. of a white, opaque, net-like gel (water content 14 wt.%, dehydration percentage 89.5 wt.%) was obtained. When this gel was immersed in 30 ml. of a pre-sterilized 0.9% saline solution for 6 hours, it absorbed water and its weight increased to 8.1 g. (water content 78 wt.%). The aforementioned yeast was not detected from this immersion solution.

Next, the net-like gel was cut into many small pieces (2 x 2 cm), then the cut pieces were washed with 40 ml. of a pre-sterilized 0.9% saline solution and the washing was observed through an optical microscope. In this observation, a small quantity of the yeast was recognized, but as a result of determination based on the turbidity of the washing, it became clear that at least 98% of the initial yeast was embedded firmly in the gel (net).

40 ml. of an alcohol preparing aqueous substrate solution comprising 5 wt.% of glucose and 60 ppm of magnesium sulfate heptahydrate was charged into a Sakaguchi flask (500 ml.), then sterilized at 120°C for 20 minutes and allowed to cool in a sterile room. Thereafter, 8 g. of the above cut pieces of the gel (immobilized yeast) were introduced therein and the flask, after putting a burnt cotton plug, was shaken in a thermostatic room at 30-33°C. As a result, after 20 hours, 2.1 wt.% of ethyl alcohol was detected from this aqueous substrate solution. Exudation of the yeast into the aqueous substrate solution was not recognized.

On the other hand, 40 ml. of the above aqueous substrate solution was added into 8 g. of a suspension containing 0.4 g. of Saccharomyces cervisiae of the same strain as the above yeast, followed by shaking in the same manner. As a result, after 20 hours, the ethyl alcohol concentration of the resulting aqueous suspension reached 2.2 wt.% (86% of theory), from which it is apparent that the yeast embedded in the gel of the present invention was kept immobilized in the gel throughout the operation of alcoholic fermentation and that is glycolysis activity (ethyl alcohol producing ability) reaches 90% of the initial unimmobilized yeast.

Example 21

86 g. powder (water content 7 wt.%) of a commercially available polyvinyl alcohol [degree of hydrolysis: 97 mol%, viscosity-average polymerization degree: 1,700, viscosity as a 4% aqueous solution: 26 cP (20°C)] was dissolved in 914 g. of water to prepare an 8.0 wt.% aqueous solution thereof (pH 6.8). 170 g. of the aqueous polyvinyl alcohol solution was sterilized with steam under pressure at 120°C for 20 minutes and then allowed to cool in a sterile room. Thereafter, 20 g. of a suspension (phosphoric acid buffer solution) containing 4 g. of Erwinia herbicola was poured into the aqueous solution followed by stirring for 7 minutes to give an aqueous suspension containing 7 wt.% of polyvinyl alcohol.

190 g. of the aqueous suspension was poured in a sterile room onto a projections-disposed plate made of polyethylene (height of projection: 4 mm, projection density: 74,000 pcs/m$^2$, shape of projection: a 1.8mm-dia. cylinder, percentage total area occupied by projections: 20%, size of the plate: 45cm x 17cm) and spread uniformly with a spatula to a thickness of 3 mm, followed by cooling (freeze-molding) at -50°C for 0.7 hour and subsequent vacuum-dehydrating for 4 hours. After thawing, 21.6 g. of a white opaque gel (net) (water content 19.9 wt.%, dehydration percentage 88.6 wt.%) was obtained. This net-like gel was immersed in 130 ml. of a pre-sterilized 0.9% saline solution for 6 hours; as a result, it absorbed water and its weight reached 86 g. (water content 80 wt.%). The net-like gel after wetting was uniformly white and translucent throughout its surface, and its apparent tensile strength reached

1 kg/cm$^2$. The aforesaid bacterium was not detected from this immersion solution and therefore it is seen that almost all quantity of the bacterium was embedded in the net-like gel.

86 g. of the above net-like gel was charged irregularly into an acrylic resin column 3cm in diameter by 60cm high, and a tyrosine preparing aqueous substrate solution (phenol 0.1%, sodium nitrite 0.2%, ammonium acetate 5%, sodium pyruvate 3%, pyridoxal phosphate 100 ppm, ethylenediamine tetraacetate 300 ppm, pH 8, 30°C) which had been sterilized at 120°C for 20 minutes was introduced from the bottom of the column at a rate of 150 ml/h. As a result, after 6 hours, the concentration of $\beta$-tyrosine [$\beta$-(p-hydroxyphenyl)alanine] in the effluent reached 630 ppm (yield: 32 mol%).

Example 22

85 g. powder (water content 6 wt.%) of a commercially available polyvinyl alcohol [degree of hydrolysis: 99.4 mol%, viscosity-average polymerization degree: 3,300, viscosity as a 4% aqueous solution: 125 cP (20°C)] was dissolved in 915 g. of water to prepare an 8 wt.% aqueous solution thereof (pH 6.9). 380 g. of this aqueous polyvinyl alcohol solution was sterilized with steam under pressure at 120°C for 20 minutes and then allowed to cool in a sterile room. Thereafter, 20 ml. of a suspension tris(hydroxymethyl)aminomethane buffer solution, pH 8.0 containing 4 g. of Lactobacillus brevis was poured into the aqueous solution followed by stirring for 7 minutes to obtain an aqueous suspension containing 7.6 wt.% of polyvinyl alcohol.

200 g. of the aqueous suspension was poured in a

sterile room onto a projections-disposed plate made of stainless steel (height of projection: 2.0 mm, projection density: 74,000 pcs/m$^2$, shape of projection: a 1.8mm-dia. cylinder, percentage total area occupied by projections: 20%, size of the plate: 48cm x 27cm) and spread uniformly with an aluminum plate (27cm x 4cm x 0.1cm) to a thickness of 1.5 mm, followed by cooling (freeze-molding) at -60°C for 0.7 hour and vacuum-dehydrating for 5 hours. After thawing, 19.4 g. of a white opaque gel (bacterial net) (water content 15 wt.%, dehydration percentage 90 wt.%) was obtained. This molded gel was immersed in 150 ml. of a pre-sterilized 0.9% saline solution for 6 hours; as a result, it absorbed water and its weight increased to 68.2 g. (water content 76 wt.%).

The net-like gel after wetting was uniformly white and translucent throughout its surface and its apparent tensile strength reached 2 kg/cm$^2$. The aforesaid bacterium was not recognized in this immersion solution, and from this fact it is seen that almost all quantity of the bacterium was embedded in the net-like gel. 68 g. of the molded gel was charged irregularly into the column described in Example 3, and a fructose preparing aqueous substrate solution (glucose 5 wt.%, manganese sulfate tetrahydrate 0.01 mol/ , 62°C, pH 6.3) which had been sterilized at 120°C for 20 minutes was introduced from the bottom of the column at a rate of 120 ml/h. As a result, after 30 hours, the effluent had a pH value of 6.7 and a fructose concentration of 2.2 wt.% (yield: 44 mol%).

Example 23

109 g. powder (water content 8.5 wt.%) of the polyvinyl alcohol used in Example 22 was dissolved in 890 g. of water to prepare 900 g. of a 10 wt.% aqueous solution thereof (pH 6.9). 390 g. of this aqueous polyvinyl alcohol solution was sampled, sterilized in the same manner as in Example 22 and then allowed to cool in a sterile room. Thereafter, 20 ml. of a suspension (culture medium) containing Streptomyces phaeochromogenes, in place of Lactobacillus brevis used in Example 22, in an amount of 1/200 of the amount used in Example 22, i.e. 0.002 g., was poured into the aqueous polyvinyl alcohol solution. 200 g. of the mixed aqueous polyvinyl alcohol - bacterium suspension was subjected to freeze-molding and dehydration in the same way as in Example 22 to yield 23 g. of a molded gel (water content 17 wt.%, dehydration percentage 89 wt.%). This molded gel was immersed in 150 ml. of a pre-sterilized 0.9% saline solution for 6 hours; as a result, it absorbed water and its weight reached 98 g. (water content 80 wt.%).

The net-like gel after wetting was uniformly white and translucent throughout its surface and its apparent tensile strength was 3 kg/cm$^2$.

The molded gel (98 g.) was charged irregularly into the column described in Example 21, and a culture solution (xylose 1%, peptone 1%, meat extract 1%, yeast extract 0.3%, common salt 0.5%, magnesium sulfate heptahydrate 0.06%, 30°C) which had been sterilized at 120°C for 15 minutes was introduced from the bottom of the column over a 24 hour period at a rate of 190 ml/h. Before and after this operation for introducing the culture solution (medium), part of the packing was sampled and observed with a scanning electron microscope; as a result, before the said operation, the bacterium was scarcely recognized in the gel, but after that operation, a bacterial colony (about 50 μm, the number of bacteria: about 300,000) was recognized in many portions in the gel.

Then, a fructose preparing aqueous substrate solution (glucose 5%, magnesium sulfate 0.01M, pH 8.3, 65°C) was introduced from the bottom of the column at a rate of 130 ml/h. As a result, after 24 hours, the fructose concentration of the effluent reached 2.1 wt.% (yield: 42 mol%) and the pH value thereof was 6.4.

When the aforesaid culture (growth) operation was omitted and the substrate solution was introduced immediately over a 24 hour period, the fructose concentration of the effluent proved to be only 0.02 wt.%.

Thus, in the present invention a live microorganism can be immobilized and therefore, as a matter of course, it can be grown in gel.


Comparative Example 7

40 g. of the mixed aqueous polyvinyl alcohol - yeast suspension obtained in Example 19 was poured onto the projections-disposed plate and spread in the same manner as in Example 19, followed by standing overnight at room temperature, to obtain 4.4 g. of a perforated, colorless, transparent film (0.07 mm thick, water content 18 wt.%). This film, when immersed in 20 ml. of tap water for 1 hour, increased in weight to 11 g.

(water content 70 wt.%) and at the same time a small amount of polyvinyl alcohol was dissolved out into the water. Moreover, the film was completely lacking in stiffness, was very weak like a wet cellophane paper and got out of shape completely to the extent that the shape of a perforated plate could no longer be recognized. It was then charged into the reaction column in the same manner as in Example 19, whereupon the column was blocked. Thus, according to the conventional method wherein a mixed aqueous suspension of polyvinyl alcohol and yeast is merely air-dried, there only is formed a weak film inferior in water resistance even if a projections-disposed plate is used.

Example 24

84 g. powder (water content 5 wt.%) of the polyvinyl alcohol used in Example 22 was dissolved in 916 g. of water to prepare an 8.0 wt.% aqueous solution thereof (pH 6.9).

Separately, 100 g. of a commercially available bentonite (powder for reagent, water content 12 wt.%) was dispersed in 1,500 g. of water to obtain a 5.5 wt.% aqueous bentonite suspension (pH 10.4), into which was added 4 ml. of 6N sulfuric acid to adjust the pH value of the suspension to 7.0.

60 g. of the above aqueous polyvinyl alcohol solution and 100 g. of the above bentonite suspension were mixed, then sterilized with steam under pressure at 120°C for 6 hours and allowed to cool in a sterile room. Thereafter, 20 g. of a suspension (phosphoric acid buffer solution, pH 7) containing 4 g. of Saccharomyces cerevisiae was added and stirring was made for 7 minutes. The polyvinyl alcohol concentration and the concentration of the suspended bentonite in the resulting aqueous suspension are 2.7 wt.% and 3.1 wt.%, respectively. On the other hand, from the results of analysis of the bentonite (powder) (X-ray diffractiometry, differential thermal analysis, identification through an electron microscope, heat dehydration, interlaminar expansion by glycerin, cation exchange: 137 meq/100g, chemical analysis: $SiO_2$ 71, $Al_2O_3$ 9, $Fe_2O_3$ 3, CaO 0.5, MgO 3, $TiO_2$ 0.5, $Na_2O$ 4, $K_2O$ 0.5, MnO 0.0, $P_2O_5$ 0.0, $H_2O$ 8.5 wt.%), its dry clay-minerals composition proves to be montmorillonite group 66, illites 1, talc 1, pyrophyllite 14 and vermiculite 1. Therefore, the concentration of three-layer type clay minerals in the above aqueous suspension is 2.6 wt.% and the amount of the clay minerals used is equal to that of polyvinyl alcohol. 180 g. of this aqueous suspension was poured in a sterile room onto a projections-disposed plate made of polyurethane rubber (height of projection: 1 mm, projection density: 74,000 pcs/$m^2$, shape of projection: a 1.8mm-dia. cylinder, percentage total area occupied by projections: 20%, size of the plate: 130cm x 25cm) and spread uniformly with a spatula to a thickness of 0.7mm, followed by cooling (freeze-molding) at -58°C for 0.7 hour and subsequent vacuum-dehydrating for 4 hours. After thawing, 17 g. of a white opaque gel (net) (water content 15 wt.%, dehydration percentage 91 wt.%) was obtained. The molded gel was immersed in 84 ml. of a pre-sterilized 0.9% saline solution for 10 hours; as a result, its weight reached 68 g. (water content 79 wt.%). The aforesaid yeast was not detected from this immersion solution. The net-like gel after wetting was uniformly white and translucent

0060052

throughout its surface, and its apparent tensile strength reached 3 kg/cm$^2$.

Then, 40 ml. of an alcohol preparing aqueous substrate solution comprising 5 wt.% of glucose and 60 ppm of magnesium sulfate heptahydrate was charged into a Sakaguchi flask (500 ml.), sterilized at 120°C for 25 minutes and then allowed to cool in a sterile room. Thereafter, 4.0 g. of the above molded gel (yeast net) was put therein and the flask, after putting a burnt cotton plug, was shaken in a thermostatic room at 28-31°C. As a result, after 12 hours, 2.1 wt.% of ethyl alcohol was detected from the substrate (aqueous solution). Exudation of the yeast into the substrate (aqueous solution) was not recognized.

On the other hand, 40 ml. of the above aqueous substrate solution was added into 4.5 g. of a suspension containing 0.4 g. of Saccharomyces cerevisiae of the same strain as the above yeast, followed by shaking in the same manner. As a result, after 12 hours, the concentration of ethyl alcohol in the resulting aqueous suspension reached 2.1 wt.% (82% of theory). Therefore, it is apparent that the yeast embedded in the net-like gel of the present invention was kept immobilized in the gel throughout the operation of alcoholic fermentation and that its glycolysis activity (ethyl alcohol producing ability) reaches 99% of the initial unimmobilized yeast.

The above commercial bentonite powder (2.0 g.) was subjected to a swelling force test according to the Pharmacopoeia of Japan; that is, 100 ml. of water was poured into a graduated measuring cylinder and the above powder was added in ten stages in such a manner that after almost all quantity of a previously added sample had precipitated the next sample was added. After
addition of the whole quantity, the cylinder was allowed to stand
for 24 hours; as a result, the apparent volume of the precipitate
was only 9 ml. far less than the defined value of 20 ml. (or more).
Likewise, with respect to the bentonite powder (6.0 g.), a gel
forming ability test was conducted according to the Pharmacopoeia
of Japan; that is, the above powder was mixed with 0.30 g. of
magnesium oxide, then the mixture was added into 200 ml. of water
in several stages, and after shaking for 1 hour, 100 ml. of the
resulting suspension was sampled and allowed to stand for 24 hours.
As a result, the amount of a transparent liquid separated as an
upper layer reached 4 ml. larger than the defined value of 2 ml.
(or less).

Thus, the commercial bentonite used in this Example
does not satisfy what is defined by the Pharmacopoeia of Japan,
but it is apparent that the said bentonite is employable in the
present invention without any trouble as previously noted.


Example 25

87 g. powder (water content 7 wt.%) of the polyvinyl
alcohol used in Example 22 was dissolved in 919 g. of water to
prepare an 8 wt.% aqueous solution thereof (pH 6.9). Separately,
120 g. of a commercial vermiculite powder (water content 9 wt.%)
was dispersed in 1,200 g. of water to obtain an 8 wt.% aqueous
suspension (pH 6.6). 190 g. of the above aqueous polyvinyl
alcohol solution and also 190 g. of the above vermiculite suspension were mixed, then sterilized with steam under pressure at
120°C for 20 minutes and allowed to cool in a sterile room.

Thereafter, 20 ml. of a suspension (phosphoric acid buffer solution, pH 7) containing 4 g. of Candida sp. was added and stirring was made for 7 minutes. The concentration of polyvinyl alcohol and that of vermiculite in the resulting aqueous suspension are each 3.8 wt.%.

On the other hand, from the results of analysis of the vermiculite (powder), its dry clay-minerals composition (wt.%) is montmorillonite group 4, illites 2, talc 3, pyrophyllite 2 and vermiculite 85 ($SiO_2$ 42, $Al_2O_3$ 19, $TiO_2$ 2, $Fe_2O_3$ 8, FeO 2, CaO 1, MgO 22, $K_2O$ 1, $Na_2O$ 1). Therefore, the concentration of three-layer type clay minerals in the above aqueous suspension is 3.5 wt.% and the amount of the clay minerals used is almost equal to that of polyvinyl alcohol.

400 g. of this aqueous suspension was poured in a sterile room onto a projections-disposed plate made of epichlorohydrin rubber (height of projection: 0.1 mm, projection density: 500,000 pcs/$m^2$, shape of projection: a 0.4mm-dia. needle, percentage total area occupied by projections: 6%, size of the plate: 355cm x 129cm) and spread uniformly with an aluminum plate (355cm x 35cm x 0.1cm) to a thickness of 0.09 mm, followed by freeze-molding at -65°C for 0.6 hour and subsequent vacuum-dehydrating for 4 hours. After thawing, 44 g. of a white opaque gel (net) (water content 22 wt.%, dehydration percentage 89 wt.%) was obtained. This molded gel was immersed in 100 ml. of a pre-sterilized 0.9% saline solution for 6 hours; as a result, its weight reached 84 g. (water content 59 wt.%). The aforesaid yeast was not detected from this immersion solution. The gel after wetting was uniformly white and translucent throughout its surface, and its apparent tresile strength reached 2 kg/cm$^2$.

50 ml. of an itaconic acid preparing aqueous substrate solution comprising 10% glucose, 0.1% ammonium chloride, 0.02% potassium primary phosphate, 0.05% magnesium sulfate heptahydrate and 0.05% yeast extract was charged into a Sakaguchi flask (500 ml.), then sterilized at 120°C for 20 minutes and allowed to cool in a sterile room. Thereafter, the above gel (yeast net) (7 g.) was placed therein and the flask, after putting a burnt cotton plug, was shaken in a thermostatic room at 24-27°C. Because of gradual decrease of the pH value of the aqueous substrate solution, the operation for neutralization (pH adjustment to 6.0) with 1N potassium hydroxide was performed twice a day. After 3 days, 2.1 wt.% of itaconic acid was recognized in the aqueous substrate solution, its yield was 29 mol%. Exudation of the yeast into the aqueous substrate solution was not recognized.

On the other hand, 5.0 ml. of the aforesaid aqueous substrate solution was added into 8 g. of a suspension (phosphoric acid buffer solution, pH 7) containing 0.4 g. of Candida yeast of the same strain as the above yeast, followed by shaking and pH adjustment in the same manner. After 5 days, the itaconic acid concentration of the aqueous substrate solution was 2.0 wt.% (yield: 28 mol%).

Therefore, it is apparent that the yeast embedded in the net-like gel of the present invention was wholly immobilized in the gel and that its itaconic acid forming activity can be regarded as being equal to that of the initial unimmobilized yeast.

Example 26

85 g. powder (water content 6 wt.%) of a commercially available polyvinyl alcohol [degree of hydrolysis: 97 mol%, viscosity-average polymerization degree: 2,200, viscosity as a 4% aqueous solution: 54 cP (20°C)] was dissolved in 914 g. of water to prepare an 8.0 wt.% aqueous solution thereof (pH 6.8).

Separately, 98 g. of illite (water content 8 wt.%, obtained in Ota City, Shimane Prefecture) was dispersed in 1,200 g. of water to obtain an 8 wt.% aqueous illite suspension.

370 g. of the above aqueous polyvinyl alcohol solution and 20 g. of the above aqueous suspension were mixed, then sterilized with steam under pressure at 120°C for 2 hours and allowed to cool in a sterile room. Thereafter, 20 ml. of a suspension (phosphoric acid buffer solution, pH 7) containing 4 g. of Gluconobacter suboxydans was added and stirring was made for 7 minutes. The concentration of polyvinyl alcohol and that of illite in the resulting aqueous suspension are each 7.3 wt.%. On the other hand, from the results of analysis of the illite (powder), it dry clay-minerals composition (wt.%) proves to be montmorillonite group 1, illites 87, talc 1, pyrophyllite 6 and vermiculite 1 ($SiO_2$ 52, $Al_2O_3$ 24, $Fe_2O_3$ 3, FeO 3, MgO 4, CaO 1, $K_2O$ 7, $Na_2O$ 1, $TiO_2$ 1, $Mn_2O_3$ 2). Therefore, the concentration of three-layer clay minerals in the above aqueous suspension is 0.36 wt.% and it is 1/20 of the concentration of polyvinyl alcohol.

41.0 g. of this aqueous suspension was poured in a sterile room onto a projections-disposed plate made of nitrile rubber (height of projection: 4mm, projection density: 3,100

pcs/$m^2$, shape of projection: a 6mm-dia. cylinder, percentage total area occupied by projections: 39%, size of the plate: 10cm x 12cm) and spread uniformly with an aluminum plate (10cm x 4cm x 0.3cm) to a thickness of 3.5 mm, followed by freeze-molding at -62°C for 0.7 hour and subsequent vacuum-dehydrating for 7 hours. After thawing, 83 g. of a net-like gel (water content 23 wt.%, dehydration percentage 80%) was obtained. When this molded gel was immersed in 400 ml. of a pre-sterilized 0.9% saline solution for 6 hours, it absorbed water and increased in weight to 223 g. (water content 71 wt.%). The gel after wetting was uniformly white and opaque and its apparent tensile strength reached 1 kg/$cm^2$. The aforesaid bacterium was not detected from this immersion solution.

223 g. of the above gel was charged irregularly in the column used in Example 2, and a D-xylose preparing aqueous substrate solution (D-arabitol 5%, magnesium sulfate heptahydrate 0.02%, potassium phosphate, buffer solution 0.06M, pH 5, 35°C) which had been sterilized at 120°C for 20 minutes was introduced from the bottom of the column at a rate of 73 ml/h. As a result, after 18 hours, the D-xylose concentration of the effluent was 3.7 wt.% (yield: 42 mol%).

CLAIMS

1.    A method of immobilizing a live microorganism which comprises the steps: preparing a 1 to 25 weight % aqueous solution of a polyvinyl alcohol having a degree of hydrolysis not less than 95 mol% and a viscosity-average polymerization degree of not less than 1,500, adding said live microorganism into said aqueous polyvinyl alcohol solution, pouring the resulting aqueous suspension into a desired shape of a vessel or a mold, freeze-molding the aqueous suspension at a temperature lower than $-6^{o}C$, vacuum-dehydrating the resulting molded article without thawing it to a dehydration percentage not lower than 5 weight %, and thawing the dehydrated article.

2.    A method according to Claim 1, in which clay minerals of a laminated structure having a three-layer type (2: 1 type) composite layer as a basic unit are suspended in said aqueous polyvinyl alcohol solution in an amount of not more than five times by weight the amount of said polyvinyl alcohol.

3.    A method according to Claim 2, in which said clay minerals are used in an amount not more than five times by weight the amount of said polyvinyl alcohol.

4.    A method according to Claim 2, in which the weight ratio of said polyvinyl alcohol to said clay minerals is in the range of 5/1 to 15/1.

5.    A method according to any one of Claims 2 to 4, in which said clay minerals principally comprise montmorillonite, vermiculite, illite, pyrophyllite, or talc.

6.    A method according to any one of Claims 2 to 5, in which said clay minerals have a particle size not larger than 0.15 mm.

7.    A method according to any preceding claim, in which the degree of hydrolysis of said polyvinyl alcohol is not less than 97 mol%.

8.    A method according to any preceding claim, in which the viscosity-average polymerization degree of said polyvinyl alcohol is in the range of 1,700 to 2,600.

9.      A method according to any preceding claim, in which the concentration of said polyvinyl alcohol in said aqueous polyvinyl alcohol solution is in the range of 7 to 15 weight %.

10.     A method according to any preceding claim, in which said freeze-moulding is carried out at a temperature lower than -15$^{o}$C.

11.     A method according to any preceding claim, in which the amount of said live microorganism added is in the range of 1/1,000 to 7 times by weight based on the amount of said polyvinyl alcohol or the total amount of said polyvinyl alcohol and said clay minerals.

12.     A method according to any preceding claim, in which said vacuum hydration is carried out up to a dehydration percentage not less than 15 weight %.

13.     A method according to any preceding claim, in which said vessel or mold is a plate having 900 to 500,000 pieces of projections per square meter.

14.     A method according to Claim 13, in which said plate is a flat plate or a curved plate.

15.     A method according to Claim 13, in which the aqueous solution is applied onto said plate so that the applied thickness is in the range of 0.01 to 5 mm.

16.     A method according to any preceding claim, in which the resulting hydrogel is further immersed in water until its water content reaches 50 to 95 weight % on a wet body basis.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | GB-A-1 556 584 (SANRAKU-OCEAN CO) *The whole document* | 1 | C 12 N 11/04 <br> C 12 N 11/08 |
| A | US-A-3 746 621 (ASAJI KONDO) *The whole document* | 1 | |
| A | GB-A-1 412 587 (GARY AIRCRAFT CORPORATION) *The whole document* | 1 | |
| A | CHEMICAL ABSTRACTS, vol. 93, no. 26, 29th December 1980, page 439, column 1, no. 246116c, Columbus Ohio (USA); L.K.ZGADZAI et al.: "Structure and thermal stability of complexes of poly(vinyl alcohol) with montmorillonites". & KOLLOIDN. ZH. 1980, 42(5), 956-60. *Abstract* | 1,4-5 | |
| A | CHEMICAL ABSTRACTS, vol. 86, no. 23, 6th June 1977, page 214, column 1, no. 167164u, Columbus Ohio (USA); & HU - A - 12 803 (MAGYAR TUDOMANYOS AKADEMIA SZEGEDI BIOLOGIAI KOZPONT BIOKEMIAI INTEZETE) (28-01-1977) *The whole document* | 1 | |

TECHNICAL FIELDS SEARCHED (Int. Cl. ³)

C 12 N

The present search report has been drawn up for all claims

| Place of search | Date of completion of the search | Examiner |
|---|---|---|
| THE HAGUE | 04-06-1982 | MARIE A.L.L. |